# EUROPEAN PATENT APPLICATION

(11) **EP 2 829 603 A1**
(43) Date of publication of application: **28.01.2015**
(21) Application number: 13769958.3
(22) Date of filing: 25.03.2013
(51) Int. Cl.: C12N 11/14, C12N 9/42, C12N 15/09

(54) **PROTEIN ALIGNMENT METHOD**

(30) Priority: 24.03.2012 JP 2012068516
(71) Applicant: Japan International Research Center for Agricultural Sciences, Tsukuba-shi Ibaraki 305-8686 (JP)
(72) Inventor: KOSUGI, Akihiko, Tsukuba-shi Ibaraki 305-8686 (JP); MORI, Yutaka, Tsukuba-shi Ibaraki 305-8686 (JP); IGARASHI, Kiyohiko, Tokyo 113-8654 (JP)
(74) Representative: Isarpatent
(86) International application number: PCT/JP2013/058609
(87) International publication number: WO 2013/146695

(57) **Abstract**

A method for aligning proteins is provided. The method for aligning the proteins (20 and 32) is capable of aligning proteins (20 and 32) on the substrate (10) comprising carbon. The proteins (20 and 32) are used as structures comprising a cellulose binding domain (22) of the cellulosome and a cohesin domain (21), and a protein (30) to be bonded to the protein (20) is used as a protein (32) having a dockerin domain (31). The cohesin domain (21) can be used as an amino-acid sequence for a cohesin domain (21) derived from various microorganisms, and a functional protein can be used as a protein structure having a microbial-species-specific dockerin domain. The protein (20) can be arrayed regularly along a graphite hexagonal-plate-shaped crystal, and any protein (32) having this dockerin domain (20) can be bonded and arranged as an active protein (32) on the graphite (10).

## Description

### Technical Field

This invention relates to a protein alignment method. Particularly, this invention relates to a protein alignment method, which can be used as a basic applied technology for nanotechnology and the like.

### Background Art

Various researches were conducted to clarify reaction that occurs on the solid-liquid interface between cellulose and enzyme. Thermophilic anaerobic bacteria (hereafter referred to as "Clostridium thermocellum), which is a subject to study by the inventors, decompose cellulose most quickly on the earth. It is known that cellulosome, which is an enzyme complex, plays a key to decompose cellulose. It has been found by the molecular genetic research conducted so far that cellulosome has a structure completely different from that of fungal enzyme. However, the mechanism of cellulose decomposition is not clear, and it is expected that the mechanism will be clarified soon.

In the Non-Patent Literature 1, it is reported that the enzyme moves on the crystalline cellulose due to the reaction between the crystalline cellulose, which is fixed on the hydrophobic surface of the graphite, and the enzyme and that monomolecular movement of fungal cellulase can be visualized by an atomic force microscope.

When protein is adsorbed nonspecifically to the graphite surface, a structural change occurs and it loses its function of protein. No active protein, which is fixed on a carbon material such as graphite and does not lose its function, has been confirmed.

### Citation List

### Non-Patent Literature

Non-Patent Literature 1: Kiyohiko Igarashi, Anu Koivula, Masahisa Wada, Satoshi Kimura, Merja Penttila and Masahiko Samejima, "High Speed Atomic Force Microscopy Visualizes Processive Movemnet of Trichoderma reesi Cellobiohydrolase I on Crystalline Cellulose", Journal of Biological Chemistry, December 25, Vol. 284, No.52,pp. 36186-36190.,2009
Non-Patent Literature 2: N. Kodera, D. Yamamoto, R. Ishikawa, and T. Ando, "Video imaging of walking myosin V by high-speed atomic force microscopy", Nature 468, 72-76, 2010.
Non-Patent Literature 3: Demain AL, Newcomb M, Wu JH., Microbiol, "Cellulase, clostridia, and ethanol", Microbiol. Mol. Biol. Rev., Vol.69, No.1., pp.124-154, March 2005
Non-Patent Literature 4: Fierobe HP, Mingardon F, Mechaly A, Belaich A, Rincon MT, Pages S, Lamed R, Tardif C, Belaich JP, Bayer EA ,"Action of designer cellulosomes on homogeneous versus complex substrates: controlled incorporation of three distinct enzymes into a defined trifunctional scaffoldin", J Biol Chem.,280(16), pp16325-16334, Epub 2005 Feb 10

### Summary of Invention

### Technical Problem

Based on the conventional knowledge, it is expected that when a scaffold protein comprising an enzyme complex (cellulosome), which helps decompose cellulose, is adsorbed nonspecifically to graphite, it loses its function as a scaffold protein.

This invention aims to provide a method to array active protein on the surface of a carbon material and the like.

### Solution to problem

When the inventors added the partial structure of cellulosome, which was prepared by a gene recombinant technology, to the graphite and observed with an atomic force microscope, it was found that multiple scaffold proteins arrayed regularly along the graphite hexagonal-plate-shaped crystals and the enzyme subunits of cellulosome were bonded to the scaffold proteins arrayed on the graphite causing their self-organization. This finding led to this invention.

This invention supplies a method for aligning proteins, comprising; arraying proteins on a carbon substrate.

In the above method, the cellulose binding domain of cellulosome, cohesin domain and dockerin domain are used to align proteins. Preferably, a structure consisting of the cellulose binding domain of cellulosome and the cohesin domain is formed, and protein containing the dockerin domain is bonded to the cohesin domain.
It is acceptable to use any cohesin domain, which has an amino acid sequence of cohesin domain derived from various microorganisms. It is also acceptable to bind the dockerin domain, which microbial species have, to the functional protein of enzymes and the like to arrange several types of protein on a substrate by using the cohesin-dockerin interaction specific to the microbial species. For the substrate, it is preferred to use mineral materials made of carbon or to use graphite.

By using the protein alignment method described in any of the above, a composite substrate, in which proteins are arrayed on a substrate made of carbon, can be obtained.

### Advantageous Effect of Invention

According to this invention, proteins can be arrayed on a substrate made of carbon. For example, by using the cohesin domain of scaffold protein of cellulosome and the dockerin domain, which the enzyme subunit of cellulosome has, proteins can be arrayed on a substrate without losing their function.

### Brief Description of Drawings

Fig. 1 shows a pattern diagram of the composite related to the embodiment of this invention.
Fig. 2 shows a pattern diagram of the partial scaffold protein related to the embodiment of this invention.
Fig. 3 shows a pattern diagram of the method for aligning scaffold proteins on a substrate surface, related to the embodiment of this invention.
Fig. 4 shows a pattern diagram of the composite related to an alternative of the protein alignment method in this invention.
Fig. 5 shows an image of a high-speed atomic force microscope; CipA, which is a scaffold protein, is arrayed on the HOPG sample disk.
Fig. 6 shows an image of a high-speed atomic force microscope; mCipA is arrayed on the HOPG sample disk.
Fig. 7 shows the bond of the scaffold protein and CelA, (a) shows a pattern diagram and (b) shows its image of a high-speed atomic force microscope.
Fig. 8 shows the bond of the scaffold protein and CelS, (a) shows a pattern diagram and (b) shows its image of a high-speed atomic force microscope.

### Reference Sign List

1, 1A: Composite
10: Substrate
10A: Surface of substrate
20: Scaffold protein
20A: Partial scaffold protein
21: Cohesin domain
22: Cellulose binding domain
23: Cohesin domain of different microbial species
30: Protein
31: Dockerin domain
32: Functional protein containing dockerin
40: Functional protein of different microbial species
41: Dockerin domain of different microbial species
42: Functional protein containing dockerin of different microbial species
CelA: endo-type cellulase
CelS: Exo-type cellulase

### Description of Embodiments

This invention is described below in detail while referring to the drawings, embodiments and specific examples.

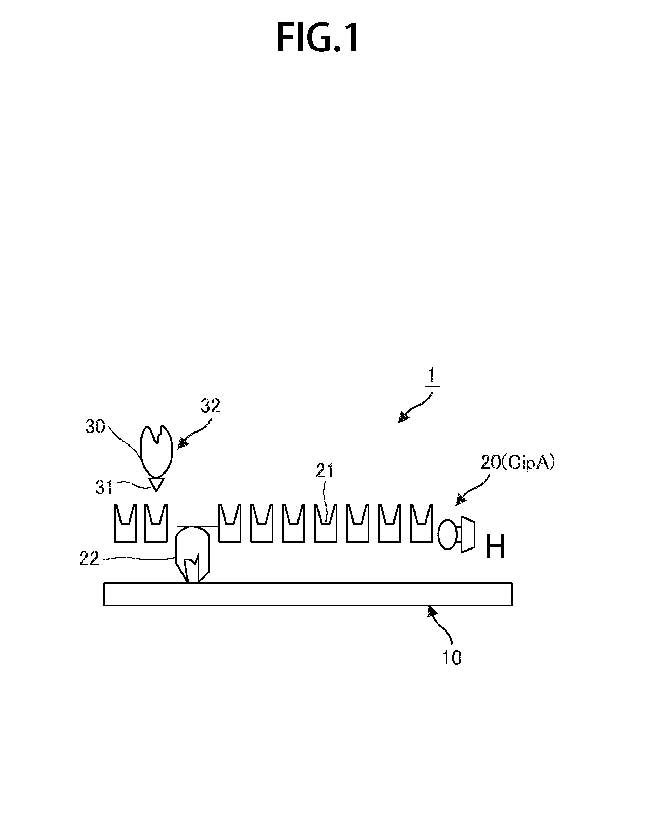

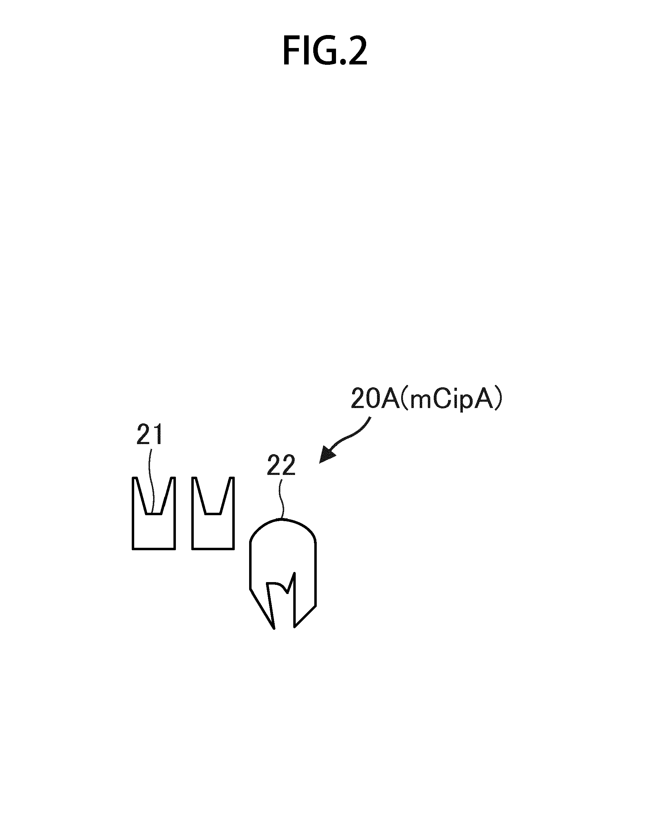

Fig. 1 shows a pattern diagram of the outlined composite 1 related to the embodiments of this invention, and Fig. 2 shows a pattern diagram of the outlined partial scaffold protein 20A related to the embodiments of this invention.
As shown by the figures, the composite 1 consists of the substrate 10 and protein containing the scaffold protein 20 such as cellulosome arrayed on the substrate 10. Namely, the protein consists of the scaffold protein 20 such as cellulosome, and the functional protein 32 containing the dockerin bonded to this scaffold protein 20. The protein structure consisting of the protein 30 and dockerin domain 31 is called the functional protein 32 containing dockerin.

Cellulosome has a scaffolding protein, which is the scaffold protein 20 for retaining cellulase and the scaffolding protein is retained on the cell surface, and cellulase is bonded to the scaffolding protein. It is reported that the scaffolding protein has the cellulase binding domain 22 and the cohesin domain 21 and that the cellulase constructing the cellulosome has the dockerin domain 31, which is bonded to the cohesin domain 21 in noncovalent bond such as hydrogen bonding (See the Non-Patent Literature 3).

Carbon materials and the like made of only carbon atoms can be used for the substrate 10. Graphite, carbon nanotube and the like can be used as carbon materials. Graphite is a native element made of carbon and has hexagonal-plate-shaped crystals. Graphite is a layered material shaped like a turtle shell. Within the in-plane of each layer, carbons are bonded with each other by strong covalent bond, but the layers are bonded with each other by weak van der Waals binding. In embodiments, graphite is used for the substrate 10 and explanation is made accordingly.

For the substrate 10, not only the graphite but any material, which has affinity with the cellulose binding domain 22, within a molecule, shown in Fig. 1 and with the cellulose binding domain 22 shown in Fig. 2 and described later, can be used. For example, the material can include cellulose.

In the composite 1 in this Embodiment, as shown in Fig. 1, the scaffold protein 20 or the partial scaffold protein 20A (see Fig. 2) is arrayed and bonded on the surface of substrate 10 through the cellulose binding domain 22.

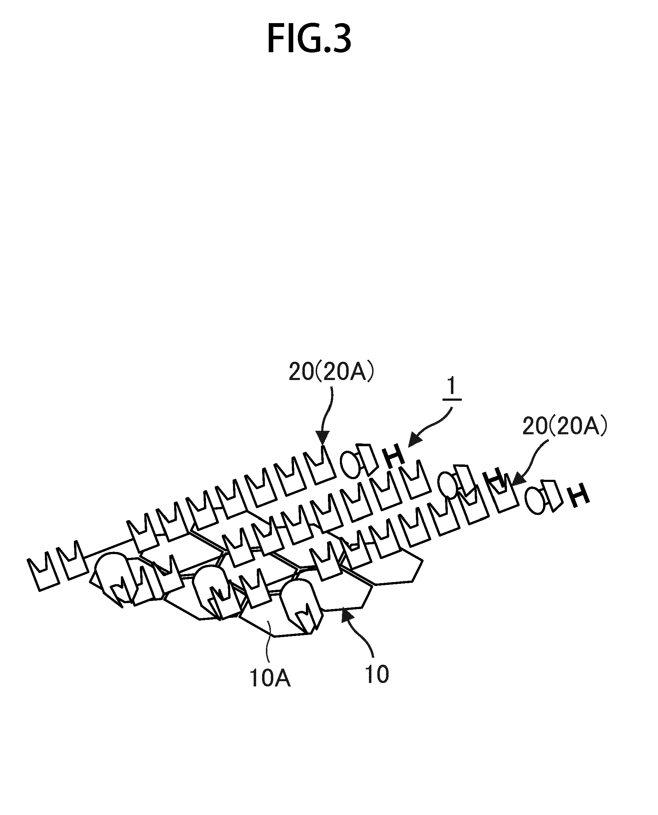

Fig. 3 shows an outline pattern diagram illustrating the method for aligning the scaffold protein 20 on the surface 10A of the substrate 10 related to the embodiments of this invention.
As shown in Fig. 3, the composite 1 is produced by adding the water solution containing multiple scaffold proteins 20 or partial scaffold proteins 20A on the surface of the face 10A, which is constructed by carbon atoms arranged like a hexagon net. The scaffold protein 20 or the partial scaffold protein 20A is arrayed on the face 10A of the substrate 10. In addition, the functional protein 32 containing the dockerin can be bonded to the scaffold protein 20 or partial scaffold protein 20A as shown in Fig. 1.

The dockerin domain 31 of the functional protein 32 containing the dockerin is bonded to the cohesin domain 21, and so the functional proteins 32 can be arrayed on the substrate 10 through a structure consisting of the cellulose binding domain 22 of cellulosome and the cohesin domain 21. For the functional protein 32 containing dockerin, a protein containing the dockerin domain 31 as its original property can be used.

It is a common property that enzyme subunits constructing the cellulosome have the dockerin domain 31 consisting of about 24 amino acids. It has been known that the dockerin domain 31 binds with the hydrophobic cohesin domain 21, which exists in the scaffold protein, in a high binding constant. The interaction between these two dockerin domain 31 and cohesin domain 21 is the basic mechanism forming the cellulosome (see Non-Patent Literature 3), and this protein-protein interaction has a relatively high binding constant.

It has been known that the above cohesin-dockerin interaction is species-specific in microorganism. Namely, the dockerin domain 31 of Clostridium thermocellum binds with the cohesin domain 21 of Clostridium thermocellum. The dockerin domain of Clostridium cellulolyticum binds with the cohesin domain of Clostridium cellulolyticum. Therefore, production of artificial cellulosome by using these properties is reported (see the Non-Patent Literature 4).

For example, as described later, the cohesin domain 21 of Clostridium thermocellum can be change into the amino acid sequence of cohesin domain of Clostridium cellulovorans, which produces cellulosome similarly. In that case, the functional protein containing a dockerin can be used as a dockerin domain derived from the same microorganism as the cohesin domain. Namely, functional protein containing the dockerin domain of Clostridium cellulovorans can be bonded to the above cohesin domain. By using this interaction of microbial-species-specific cohesin-dockerin domains, two or more, several types of proteins can be arranged and arrayed without losing their original function.

### (Example of scaffold protein)

A specific example of scaffold protein 20 is described. As scaffold protein 20, an enzyme cloned from the one contained in cellulosome (enzyme complex) can be used. This enzyme has as its original property the dockerin domain 31, which binds with the cohesin domain 21 that constructs the scaffold protein 20.

One example of the scaffold protein 20 is CipA, and this CipA (196kDa) has nine cohesin domains 21 and one cellulose binding domain 22 as shown in Fig. 1. The cellulose binding domain 22 is arranged between the second cohesin domain 21 and the third cohesin domain 21. For these cohesin domains 21, the scaffold protein derived from microorganism, for example, Clostridium thermocellum can be used.

Instead of the scaffold protein 20, the scaffold protein 20A, which is a part of it, can be used. The partial scaffold protein 20A shown in Fig. 2 is mCipA (its molecular weight is 53kDa), and this mCipA is a partial structure of CipA from the N terminal of CipA to the cellulose binding domain 22. mCipA has the cellulose binding domain 22 and two cohesin domains 21 as shown in Fig. 2. These scaffold protein 20 and partial scaffold protein 20A are produced by a recombination technology.

### (Dockerin domain)

In addition, similarly the above dockerin domain derived from microorganism can be used as the dockerin domain 31, which has affinity with the cohesin domain 21 of the scaffold protein 20. For the functional protein 32 containing the dockerin having the dockerin domain 31, any of enzyme, antibody and labeled compound can be used.

For the dockerin domain 31 in this Embodiment, the one derived from microorganism same as the cohesin domain 21 can be used. For example, for the scaffold protein 20 and the partial scaffold protein 20A, which is a part of it, the following proteins derived from microorganism can be used.
Clostridium cellulovorans
Clostridium cellulolyticum
Clostridium josui
Clostridium acetobutylicum
Clostridium cellobioparum
Clostridium papyrosolvens
Ruminococcus albus
Ruminococcus flavefaciens
Acetivibrio cellulolyticus
Bacteroides cellulosolvens
Butyrivibrio fibrisolvens

### (One example of method for producing composite)

One example of the method for arraying the scaffold protein 20 and the functional protein 32 containing the dockerin, which binds with this scaffold protein, is described below.

Firstly, water solution containing the scaffold protein 20 is added on the substrate 10 made of graphite. Then multiple scaffold proteins 20 are arrayed on the graphite surface.

Subsequently, as the functional protein 32 containing the dockerin, the water solution containing the enzyme 30 is added on the substrate 10. Then, the dockerin domain 31 of the enzyme 30 binds with the cohesin domain 21 of the scaffold protein 20 and self-organizes on the substrate 10.

Thus, the enzyme 30 can be arrayed on the substrate 10, which is made of carbon material and the like, through the dockerin domain 31. The temperature can be adjusted according to the enzyme 30 used.

According to the protein alignment method of this invention, for example, a partial structure of cellulosome can be produced by using a protein recombination technology, and by adding it to the graphite, multiple scaffold proteins 20 can be arrayed regularly along the hexagonal-plate-shaped crystals of the graphite. In addition, the enzyme subunits of cellulosome can be bonded to the scaffold proteins 20 arrayed on the graphite, causing their self-organization.

The technology for arraying proteins on the substrate 10 made of carbon materials such as graphite can be used for, for example, biochip, biosensor, biological device, microarray, chromatographic carrier, immobilized polymer, immobilized enzyme, taste sensor, prevention of nonspecific adsorption of protein, biochip for medical material, element for carrying protein drug, protein separation, and manufacture of functionalized molecule.

Specifically, the scaffold protein 20 is arrayed on the substrate 10 made of carbon material. Subsequently, by using, needless to say, the cellulosome enzyme subunit, which has the dockerin domain 31 as its original property, or proteins and the like, to which the dockerin domain 31 is artificially attached as its function by gene recombination, self-organization can be performed by the cohesin-dockerin interaction with the scaffold protein 20 arrayed on the substrate 10.

For the above proteins, hydrolytic enzyme, oxygen reduction enzyme, transfer enzyme, addition-elimination enzyme (lyase), isomerase, synthesis enzyme (ligase) and proteins, which have an enzyme function and EC number, such as those catalyzing epimerization reaction, intramolecular rearrangement and racemization reaction can be used. In addition, as protein, an antibody, labeled protein and chimera protein, in which a chemical agent is attached directly to the dockerin domain 31 by using conjugate, can be used according to the situation.

In addition to the above proteins, it is acceptable to use a low-molecular compound, labeled compound having radio isotope, and labeled compound having stable isotope.
The dockerin domain 31 can be directly labeled, or the dockerin domain 31, to which a compound having a labeling function is attached, can be used. Therefore, any form can be used as far as it has the dockerin domain 31 and can interact with the scaffold proteins 20 arrayed on the substrate 10 made of carbon and can align the proteins at least.

### (Alternative of protein alignment method)

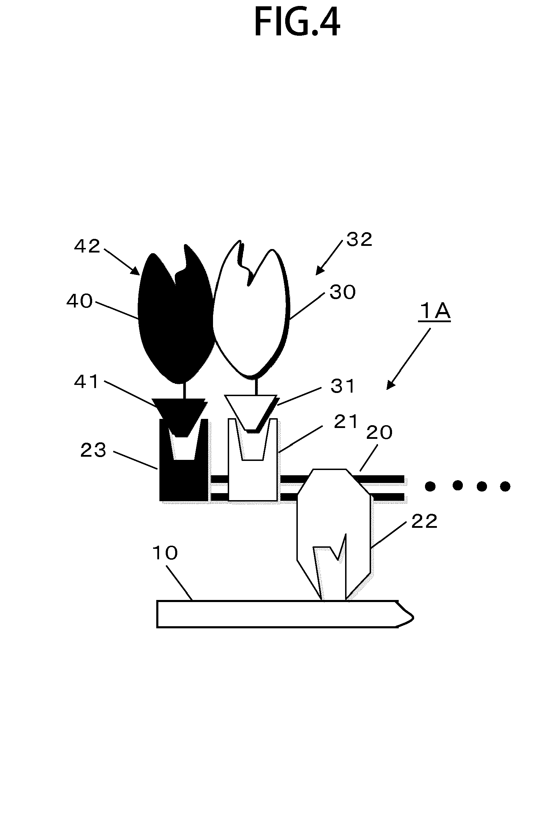

Fig. 4 shows a pattern diagram of the composite 1A related to an alternative of the protein alignment method of this invention. As shown in this figure, the composite1A differs from the composite 1 shown in Fig. 1 in that the cohesin domain 23 of different microbial species is additionally bonded to the scaffold protein 20, which is bonded to the substrate 10 through the cellulose binding domain 22. Furthermore, the composite 1A differs from the composite 1 shown in Fig. 1 in that the functional protein 42 containing the dockerin is bonded to the cohesin domain 23 of different microbial species. The functional protein 42 containing this dockerin consists of the protein 40 of the same microbial species as that of the cohesin domain 23 and the dockerin domain 41.

It has been known that the above cohesin-dockerin interaction is specific to microbial species (see Non-Patent Literature 3). Namely, the dockerin domain 31 of Clostridium thermocellum binds with the cohesin domain 21 of Clostridium thermocellum, and the dockerin domain 41 of Clostridium cellulovorans binds with the cohesin domain 23 of Clostridium cellulovorans. Therefore, production of artificial cellulosome using these properties is reported (Non-Patent Literature 4).

By using this technology, when the cohesin domain 21 of the scaffold protein 20 bonded to the composite 1 in Fig. 1 is made to have the amino acid sequence of the cohesin domain 23 derived from different microbes and also the protein 40 such as enzyme is made to have the dockerin domain 33 of the microorganism derived from the cohesin domain 23, several types of different functional proteins 32 and 42 can be arranged (see Non-Patent Literature 4).
Thus, by making the cohesin domain 21 to have the amino acid sequence of the cohesin domain 23 derived from different microbial species, also making the protein 40 such as enzyme to have the dockerin domain 41 specific to its microbial species and using the microorganism-specific cohesin-dockerin interaction, different proteins 32 and 42 can be arranged on the scaffold protein 20.

For example, in the Non-Patent Literature 4, chimeric scaffold protein is produced by using the cohesin domain of Clostridium thermocellum, the cohesin domain of Clostridium thermocellum and the cohesin domain of R. flavefaciens. In addition, proteins can be arranged in an arbitrary place due to the cohesin-dockerin interaction species-specific to its chimeric scaffold protein by using the cellulase (family 9) derived from Clostridium thermocellum and having a dockerin domain, the cellulase (family 8) derived from C. cellulolyticum and having a dockerin domain, and the cellulase (family 9) derived from R. flavefaciens and having a dockerin domain. Furthermore, this invention is described in detail below by using examples. However, this invention is not limited to the following examples.

### Example

The model of the recombinant protein produced and used in this invention is shown in Fig. 1. The genome DNA from Clostridium thermocellum was extracted in the following procedure.
After Clostridium thermocellum was cultured in a BM7CO-CB culture medium containing 0.5% cellobiose, it was subjected to centrifugal separation at 4°C and 10,000 revolutions for five minutes to collect the fungus body.

### (Culturing of Clostridium thermocellum)

The Clostridium thermocellum JK-S14 strain (NITE BP-627) was cultured in a BM7CO-CB culture medium containing 10g/L of fine crystal cellulose at 60°C for four days.

Subsequently, to subject the obtained fungus body to bacteriolysis, 10% SDS (sodium lauryl sulfate) was added so that its final concentration became 0.5%, the proteinase K (1mg/mL) solution was also added so that its final concentration became 5 µg/mL, and reaction was performed at 37°C for one hour.
In addition, 10% cetyltrimethylammonium bromide - 0.7 M (mole) sodium chloride solution was added so that its concentration became 1%, and reaction was performed at 65°C for ten minutes. Subsequently, the equal amount of chloroform isoamyl alcohol solution was added, the solution was stirred well, and it was subjected to centrifugal separation of 15,000 revolutions for five minutes to obtain the water layer.

The equal amount of mixture of phenol, chloroform and isoamyl alcohol was added to the above water layer, and it was stirred and the subjected again to centrifugal separation of 15,000 revolutions for five minutes to obtain the water layer. Isopropanol 0.6 times its volume was added to this water layer and the genome DNA was separated out and the genome DNA was prepared by performing centrifugal separation again. This genome DNA was washed with 70% ethanol and dried.

The composition of the prepared BM7CO-CB culture medium was 1.5 g/L of potassium dihydrogen phosphate, 2.9 g/L of dibasic potassium phosphate, 2.1 g/L of urea, 6.0 g/L of yeast extract, 4 g/L of sodium carbonate, 0.05 g/L of cysteine hydrochloride, and 0.2 mL of mineral solution (MgCl₂/6H₂O 5g, CaCl₂/2H₂O 0.75g and FeSO₄/6H₂O 0.0063g were solved in 4 mL of water). In addition, cellobiose was added as a carbon source to the culture medium so that its final concentration became 5g/L. The final culture medium pH was adjusted around 7.0.

Using the above prepared genome DNA, CipA was designed and synthesized from the oligonucleotide primer CipA (SEQUENCE No.1: AGTCA GAGCTCC GCC ACA ATG ACA GTCGAG) and CipAR (SEQUENCE No. 2: AGTCACTCGAGTACAGGCTGTGTTGATGG), mCipA was designed and synthesized from the oligonucleotide primer mCipAF (SEQUENCE No. 3: AGTCA GAGCTCC GCC ACA ATG ACA GTCGAG) and mCipAR (SEQUENCE No. 4: AGTCA CTCGAG TACAGGCTGTGTTGATGG), CelA was designed and synthesized from oligonucleotide primer CelAF (SEQUENCE No. 5: AGTCA GAGCTCC GCA GGT GTG CCT TTT AAC) and CelAR (SEQUENCE No. 6: AGTC AAGCTT ATAAGGTAGGTGGGGTATG - 3), and CelS was designed and synthesized from oligonucleotide primer CelSA (SEQUENCE No. 7: AGTC GAGCTCC GGT CCT ACA AAG GCACCTA) and CelSR (SEQUENCE No. 8: AGTCA GCTGAGC GTTCTTGTACGGCAATGT). In PCR, which is a method for amplifying genes, about 5.5 kilobase long double-strand amplified DNA sequence was obtained. The amplified CipA gene sequence is shown in the SEQUENCE No. 9.

The designed oligonucleotide primers CipF and CipAR are attached with the restriction enzyme site Sacl and the Xhol site to insert them into E coli expression vector.
Similarly, oligonucleotide primer mCipAR is attached with the restriction enzyme site Xhol site. Oligonucleotide primer CelAF, CelAR. CelS, oligonucleotide primer CelSA and CelSR are attached with restriction enzyme site Sacl and Hind III, SacI and Bpu11021.

CipA gene sequence, which is the scaffold protein 20 of Clostridium thermocellum, CelA gene sequence and CelS gene sequence can be obtained through the website (http://www.Ncbi.nlm.nih.gov/) of the National Center for Biotechnology Information (NBIC)(CipA GenBank accession No.: CAA91220.1), (CelA GenBank accession No.: K03088), and (CelS GenBank accession No. L06942.1).

In PCR, 16srRNA gene was amplified by the ExTaq DNA polymerase (Takara BIO Inc.) The PCR condition was thirty cycles of 98°C for one minute, 55°C for one minute and 72°C for four minutes, and amplification was performed in this condition.

After the amplified band was checked by 0.8% agarose gel electrophoresis, the PCR products were refined by the QIAGEN PCR refining kit (QIAGEN GmbH). Using the above restriction enzyme (Takara BIO Inc.), the refined PCR products were subjected to restriction enzyme treatment at 37°C overnight.

The restriction-enzyme-treated PCR product was subjected to the 0.8% agarose gel electrophoresis again to separate the restriction enzyme decomposition product, and the intended band was cut from the gel and refined by the gel extraction kit (QIAGEN GmbH).

The pET22b expression vector (Merck KGaA) was used to express the CipA gene in E coli. This vector was so designed that 6-residue histidine tag was fused to the N-terminal side of the intended protein to be expressed. The pET19b (Merck KGaA) expression vector was used for expressing CelS in E coli. In this case, restriction enzyme treatment was performed at 37°C overnight using Sacl and Bpu1102. After the restriction enzyme treatment was performed, further treatment with Alkaline Phosphatase (Takara BIO Inc.) at 50°C for one hour was performed to dephosphorylate the restriction enzyme cutting site. After extraction of phenol chloroform was repeated and Alkaline Phosphatase was deactivated, ethanol precipitation treatment was performed, and the restriction-enzyme-treated pET19b expression vector was collected.

To construct CipA expression vector, mCipA expression vector, CelA expression vector and CelS expression vector, the above restriction-enzyme-treated pET22b and pET19b expression vectors were incubated with the T4 ligase (Takara BIO Inc.) at 16°C overnight and their fragments were connected.
Subsequently, the above expression vectors CipA-pET22b, mCipA-pET22b, CelA-pET22b and CelS-pET19b were transformed into E coli JM109 and cultured at 37°C overnight in a Luria-Bertani culture medium (called LB culture medium) containing 50 µg/mL of ampicillin sodium and 1.5% agar.

The composition of the LB culture medium is shown below.
Bacto Peptone 1g/L
Sodium chloride 1g/L
Yeast extract 1g/L
The final culture medium pH was adjusted to around 7.0.

The clone having the intended gene fragment was selected from the grown colony. For the selection, the expression vectors were extracted from the E coli clone using the plasmid extraction kit (QIAGEN GmbH). After extracting the expression vectors, with the above primer, the DNA sequence was analyzed by BigDye (registered trademark) Terminator v3.1 (Applied Biosystems), PRISM (registered trademark) 3100 Genetic Analyzer (Applied Biosystems) or PRISM (registered trademark) 3700 DNA Analyzer (Applied Biosystems).

To check whether the gene sequence was correct, homology search was performed on the website of the National Center for Biotechnology Information (NBIC) using the obtained DNA sequence data. To express a large quantity of respective proteins, the expression vectors having the correct gene sequence were transformed into the E coli BL21 (Merck KGaA) again and the clones of E coli expressing a large quantity of protein were obtained.

To obtain CipA, mCipA, CelA and CelS, the E coli BL21 having respective expression vectors were cultured at 37°C for four hours in a LB culture medium containing ampicillin sodium. After the culturing process, isopropyl-D-thiogalactopyranoside was added in concentration of 1 mM and further cultured for twelve hours.

Respective E coli BL 21 (DE3) was subjected to centrifugal separation (8,000 revolutions, 4°C and 10 minutes), and the E coli was collected. The collected E coli was frozen at -80°C overnight, suspended in the bacteriolysis buffer solution (50 mM of phosphate buffer solution, 300 mM of sodium chloride, 10 mM of imidazole, and pH 8.0) and then shattered in ice by a sonicator. After the obtained bacteriolysis suspension was subjected to centrifugal separation to separate the transparent lysis solution from the precipitated unshattered fungus body, only the transparent lysis solution was collected and filtered by a 0.45 µm filter.

The His-tag fused protein was obtained from the lysis solution through the nickel agarose gel column (Ni-NTA agarose gel QIAGEN GmbH). In addition, the eluted recombinant protein was refined through the desalting column (Bio-Rad Laboratories, Inc.) After refined proteins were diluted with distilled water as needed, amounts of respective protein is measured by the BCA- protein measuring kit (Thermo Scientific). The analytical curve of protein was prepared by using the bovine serum albumin.

The CipA gene sequence and its amino-acid sequence are shown in the SEQUENCE No. 9 and 10, respectively.

The gene sequence and the amino-acid sequence of mCipA, which is a partial structure of CipA of Clostridium thermocellum, are shown in the SEQUENCE No. 11 and 12, respectively.

The gene sequence and the amino-acid sequence of the typical cohesin domain 21, which CipA of Clostridium thermocellum has, are shown in the SEQUENCE No. 13 and 14, respectively.

The gene sequence, which codes the amino-acid sequence of the cellulose binding domain 22 (CBM) of Clostridium thermocellum, and the gene sequence of the cellulose binding domain 22 (CBM) of Clostridium thermocellum are shown in the SEQUENCE No.15 and 16, respectively.

The gene sequence and amino-acid sequence of CelA are shown in the SEQUENCE No. 18 and 19.

The gene sequence and amino-acid sequence of CelS are shown in the SEQUENCE No. 20 and 21, respectively.

The gene sequence and amino-acid sequence of dockerin domain 31 of CelA are shown in the SEQUENCE No. 21 and 22, respectively.

The gene sequence and amino-acid sequence of dockerin domain 31 of CelS are shown in the SEQUENCE No. 23 and 24, respectively.

Fig. 1 shows the protein domain structure in a pattern diagram based on the above amino-acid sequence. Observation was conducted by a high-speed atomic force microscope developed by Kodera at al. (see the Non-Patent Literature 2).

For observation by a high-speed atomic force microscope, the BL-AC10EGS-A2 small cantilever (Olympus Corporation) and the Nano Live Version (Olympus Corporation) were used.

For the graphite used as a base, the highly oriented pyrolytic graphite (called HOPG) was used. The less than 0.1mm thick HOPG was attached to a cylindrical glass stage (2mm high and 1.5mm diameter), and a Scotch tape was pressed to ensure the flatness of the HOPG surface.

The respective proteins of highly refined CipA, mCipA, CelA and CelS were prepared by 50 mM of acetic acid buffer solution (pH6.0) to 0.2 µM to 20 µM to use them for the above high-speed atomic force microscope.

About 2 µM of the above CipA and mCipA were added on the HOPG, and one to four frames of high-speed atomic force microscope image were taken within one second. Analysis of the high-speed atomic force microscope images was conducted by using the IGOR pro (WaveMetrics). All the high-speed atomic force microscope images were treated by a 2 x 2 pixel averaging filter to reduce noise spikes in the high-speed atomic force microscope.

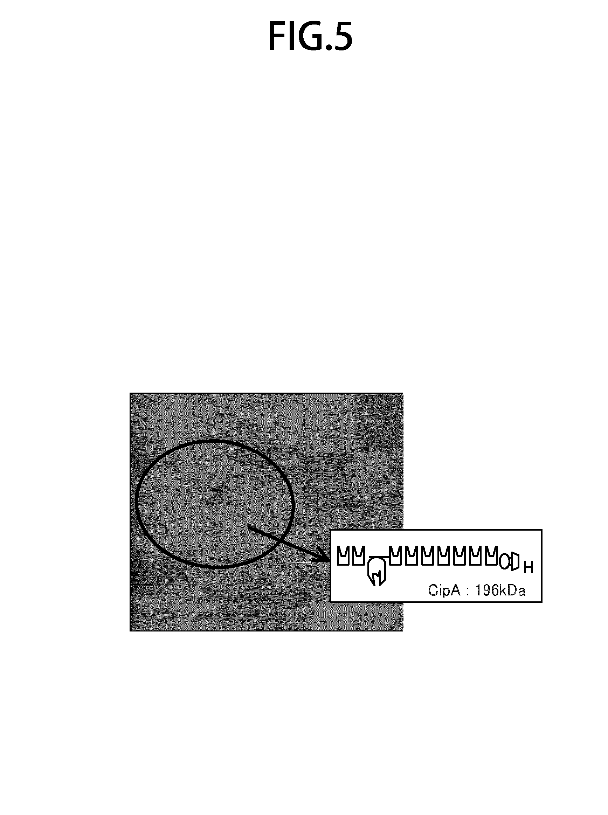

Fig. 5 shows a high-speed atomic force microscope image taken when CipA, which is the scaffold protein 20, was arrayed on the HOPG sample disk. As clearly shown by this figure, CipA was arrayed along the hexagonal carbon grid of the HOPG disk.

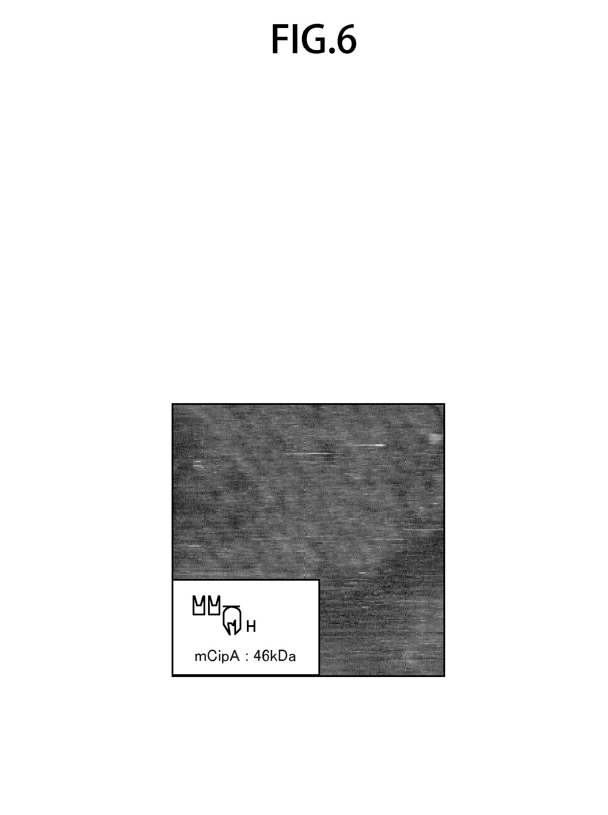

mCipA was also added to the HOPG sample disk same as CipA was done to check whether even a partial domain of CipA would be arrayed. Fig. 6 shows a high-speed atomic force microscope image taken when mCipA was arranged on the HOPG sample disk. As clearly shown by this figure, mCipA was arrayed along the hexagonal carbon grid of the HOPG disk same as CipA shown in Fig. 5.

### (Bonding of scaffold protein and functional protein)

Usually, when non-specific adsorption occurs to protein, a structural change is caused and protein loses its function. If CipA was adsorbed non-specifically to HOPG, it was expected that the scaffold protein 20 would lose its function. Two enzyme subunits CelA and CelS were prepared and a bonding test was conducted to confirm whether the enzyme subunit of cellulosome could bind with CipA arranged on the HOPG and could perform self organization.

As similar to described before, 2 µL of CelA and CelS were added on the HOPG sample disk shown before, where mCipA was arrayed, and one to four frames of high-speed atomic force microscope image were taken within one second. Analysis of the high-speed atomic force microscope images was conducted by using the IGOR pro (WaveMetrics) same as the above.

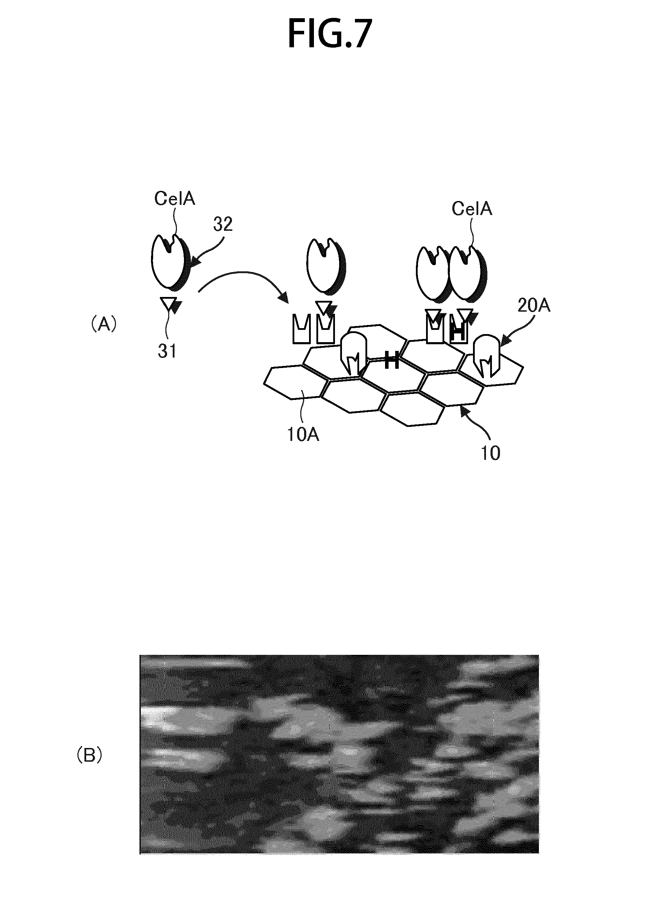

Fig. 7 shows the bond between the scaffold protein 20 and CelA. (a) shows a pattern diagram of the bond, and (b) shows a high-speed atomic force microscope image.

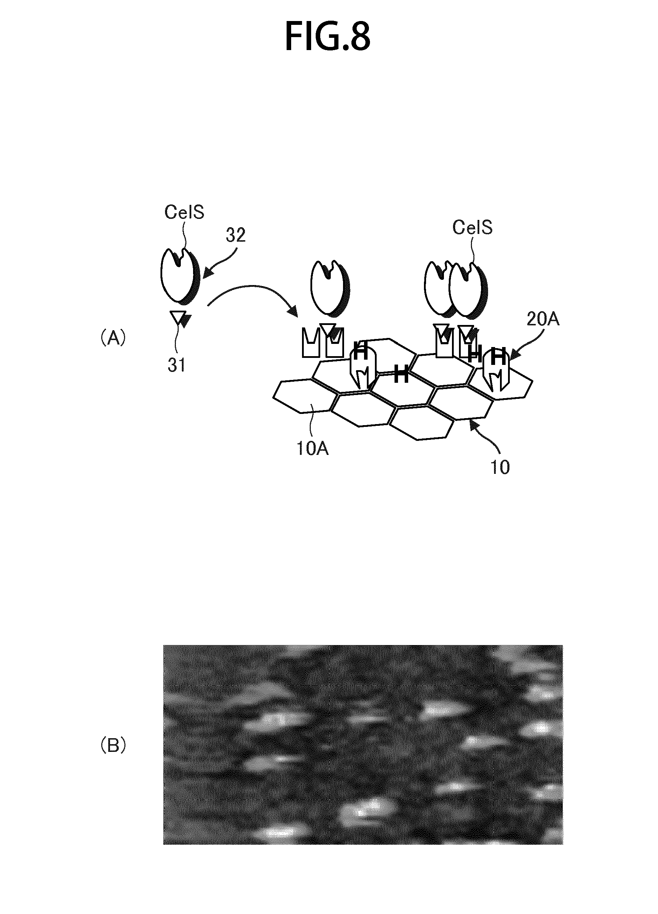

Fig. 8 shows the bond between the scaffold protein 20 and CelS; (a) shows a pattern diagram of the bond, and (b) shows a high-speed atomic force microscope image.

What are bright in yellow in the high-speed atomic force microscope images of Figs. 7 and 8 are CelA and CelS enzyme proteins, and it was shown that they were interacting with mCipA, which was the partial scaffold protein 20A arrayed regularly on the HOPG sample disk.

Based on the results shown in above Figs. 7 and 8, it was understood that non-specific adsorption did not occur in the scaffold protein 20 of CipA, that the protein was arrayed regularly while keeping its structure without losing its function as a protein, and that the cohesin domain 21 of the scaffold protein (20) interacted with the dockerin domain 31 possessed by the protein, self-organizing a cellulosome structure.

Therefore, by using the above method, not only enzymes but also desired proteins such as the functional protein 32, which contains a antibody and dockerin, can be arrayed on the surface of substrate 10 without losing their function.

This invention is not limited to the above examples, but can be used in various different forms within the scope of the invention indicated in the patent claim, and it is needless to say that such usage is also included within the scope of this invention.

### Accession Number

NITE BP-627 (Clostridium thermocellum JK-S14 strain)

### Depository Designation

Name of deposit institution: NPMD National Institute of Technology and Evaluation NITE Patent Microorganisms Depositary (NPMD)
Address of deposit institution: 2-5-8 Kazusa-Kamatari, Kisarazu, Chiba, 292-0818 Japan
Deposition date: August 12, 2008
Deposit No. NPMD NITE BP627

## Claims

1. A method for aligning proteins, comprising; arraying proteins on a carbon substrate.

2. The method for aligning proteins according to claim 1, wherein a cellulose binding domain, a cohesin domain, and a dockerin domain derived from cellulosome are used for aligning the proteins.

3. The method for aligning proteins according to claim 1 or 2, comprising arraying a structure consisting of the cellulose binding domain of cellulosome and the cohesin domains, and
combining a protein having the dockerin domain with the cohesin domain.

4. The method for aligning proteins according to claim 2, wherein the cohesin domains have amino-acid sequences of cohesin domains derived from several different microbial species, protein such as enzymes are made to have a dockerin domain, which the microbial species have, and the cohesin-dockerin interaction specific to the microbial species is used to arrange several types of protein.

5. The method for aligning proteins according to claim 1, wherein the substrate is made of a mineral material including carbon.

6. The method for aligning proteins according to claim 1 or 5, wherein the substrate is made of graphite.

7. A composite substrate made of carbon, on which the proteins are arrayed by one of the protein alignment methods according to claim 1 to 6.


SEQUENCE LISTING

<110>  Japan International Research Center for Agricultual Sciences

<120>  method for arranging protein

<130>  P3810UNV

<160>  24

<170>  PatentIn version 3.1

<210>  1
<211>  30
<212>  DNA
<213>  Clostridium thermocellum

<400>  1
agtcagagct ccgccacaat gacagtcgag                                        30


<210>  2
<211>  29
<212>  DNA
<213>  Clostridium thermocellum

<400>  2
agtcactcga gtacaggctg tgttgatgg                                         29


<210>  3
<211>  30
<212>  DNA
<213>  Clostridium thermocellum

<400>  3
agtcagagct ccgccacaat gacagtcgag                                        30


<210>  4
<211>  29
<212>  DNA
<213>  Clostridium thermocellum

<400>  4
agtcactcga gtacaggctg tgttgatgg                                         29


<210>  5
<211>  30
<212>  DNA
<213>  Clostridium thermocellum

<400>  5
agtcagagct ccgcaggtgt gccttttaac                                       30


<210>  6
<211>  29
<212>  DNA
<213>  Clostridium thermocellum

<400>  6

agtcaagctt ataaggtagg tggggtatg                                          29

<210>  7
<211>  30
<212>  DNA
<213>  Clostridium thermocellum

<400>  7
agtcgagctc cggtcctaca aaggcaccta                                         30

<210>  8
<211>  30
<212>  DNA
<213>  Clostridium thermocellum

<400>  8
agtcagctga gcgttcttgt acggcaatgt                                         30

<210>  9
<211>  5573
<212>  DNA
<213>  Clostridium thermocellum

<400>  9
   atgagaaaag tcatcagtat gctcttagtt gtggctatgc tgacgacgat ttttgcggcg      60
   atgataccgc agacagtatc ggcggccaca atgacagtcg agatcggcaa agttacagca    120
   gccgttggat caaaagtaga aatacctata accctgaaag gagtgccatc caaaggaatg    180
   gccaattgcg acttcgtatt gggttatgat ccaaatgtgc tggaagtaac agaagtaaaa    240
   ccaggaagca taataaaaga tccggatcct agcaagagct ttgatagcgc aatatatccg    300
   gatcgaaaga tgattgtatt tctgtttgca gaagacagtg gaagaggaac gtatgcaata    360
   actcaggatg gagtatttgc aacaattgta gccactgtca aatcagctgc agcggcaccg    420
   attactttgc ttgaagtagg tgcatttgcg gacaacgatt tagtagaaat aagcacaact    480
   tttgtcgcgg gcggagtaaa tcttggtagt tccgtaccga caacacagcc aaatgttccg    540
   tcagacggtg tggtagtaga aattggcaaa gttacgggat ctgttggaac tacagttgaa    600
   atacctgtat atttcagagg agttccatcc aaaggaatag caaactgcga ctttgtgttc    660
   agatatgatc cgaatgtatt ggaaattata gggatagatc ccggagacat aatagttgac    720
   ccgaatccta ccaagagctt tgatactgca atatatcctg acagaaagat aatagtattc    780
   ctgtttgcgg aagacagcgg aacaggagcg tatgcaataa ctaaagacgg agtatttgca    840
   aaaataagag caactgtaaa atcaagtgct ccgggctata ttactttcga cgaagtaggt    900
   ggatttgcag ataatgacct ggtagaacag aaggtatcat ttatagacgg tggtgttaac    960
   gttggcaatg caacaccgac caagggagca acaccaacaa atacagctac gccgacaaaa   1020
   tcagctacgg ctacgcccac caggccatcg gtaccgacaa acacaccgac aaacacaccg   1080
   gcaaatacac cggtatcagg caatttgaag gttgaattct acaacagcaa tccttcagat 1140
   actactaact caatcaatcc tcagttcaag gttactaata ccggaagcag tgcaattgat 1200
   ttgtccaaac tcacattgag atattattat acagtagacg gacagaaaga tcagaccttc 1260
   tggtgtgacc atgctgcaat aatcggcagt aacggcagct acaacggaat tacttcaaat 1320
   gtaaaaggaa catttgtaaa aatgagttcc tcaacaaata acgcagacac ctaccttgaa 1380
   ataagcttta caggcggaac tcttgaaccg ggtgcacatg ttcagataca aggtagattt 1440
   gcaaagaatg actggagtaa ctatacacag tcaaatgact actcattcaa gtctgcttca 1500
   cagtttgttg aatgggatca ggtaacagca tacttgaacg gtgttcttgt atggggtaaa 1560
   gaacccggtg gcagtgtagt accatcaaca cagcctgtaa caacaccacc tgcaacaaca 1620
   aaaccacctg caacaacaaa accacctgca acaacaatac cgccgtcaga tgatccgaat 1680
   gcaataaaga ttaaggtgga cacagtaaat gcaaaaccgg gagacacagt aaatatacct 1740
   gtaagattca gtggtatacc atccaaggga atagcaaact gtgactttgt atacagctat 1800
   gacccgaatg tacttgagat aatagagata aaaccgggag aattgatagt tgacccgaat 1860
   cctgacaaga gctttgatac tgcagtatat cctgacagaa agataatagt attcctgttt 1920
   gcagaagaca gcggaacagg agcgtatgca ataactaaag acggagtatt tgctacgata 1980
   gtagcgaaag taaaatccgg agcacctaac ggactcagtg taatcaaatt tgtagaagta 2040
   ggcggatttg cgaacaatga ccttgtagaa cagaggacac agttctttga cggtggagta 2100
   aatgttggag atacaacagt acctacaaca cctacaacac ctgtaacaac accgacagat 2160
   gattcgaatg cagtaaggat taaggtggac acagtaaatg caaaaccggg agacacagta 2220
   agaatacctg taagattcag cggtatacca tccaagggaa tagcaaactg tgactttgta 2280
   tacagctatg acccgaatgt acttgagata atagagatag aaccgggaga cataatagtt 2340
   gacccgaatc ctgacaagag ctttgatact gcagtatatc ctgacagaaa gataatagta 2400
   ttcctgtttg cggaagacag cggaacagga gcgtatgcaa taactaaaga cggagtattt 2460
   gctacgatag tagcgaaagt aaaatccgga gcacctaacg gactcagtgt aatcaaattt 2520
   gtagaagtag gcggatttgc gaacaatgac cttgtagaac agaagacaca gttctttgac 2580
   ggtggagtaa atgttggaga tacaacagaa cctgcaacac ctacaacacc tgtaacaaca 2640
   ccgacaacaa cagatgatct ggatgcagta aggattaaag tggacacagt aaatgcaaaa 2700
   ccgggagaca cagtaagaat acctgtaaga ttcagcggta taccatccaa gggaatagca 2760
   aactgtgact ttgtatacag ctatgacccg aatgtacttg agataataga gatagaaccg 2820
   ggagacataa tagttgaccc gaatcctgac aagagctttg atactgcagt atatcctgac 2880
   agaaagataa tagtattcct gtttgcggaa gacagcggaa caggagcgta tgcaataact 2940
   aaagacggag tatttgctac gatagtagcg aaagtaaaat ccggagcacc taacggactc 3000
   agtgtaatca aatttgtaga agtaggcgga tttgcgaaca atgaccttgt agaacagaag 3060
   acacagttct ttgacggtgg agtaaatgtt ggagatacaa cagaacctgc aacacctaca 3120
   acacctgtaa caacaccgac aacaacagat gatctggatg cagtaaggat taaagtggac 3180
   acagtaaatg caaaaccggg agacacagta agaatacctg taagattcag cggtatacca 3240
   tccaagggaa tagcaaactg tgactttgta tacagctatg acccgaatgt acttgagata 3300
   atagagatag aaccgggaga cataatagtt gacccgaatc ctgacaagag ctttgatact 3360
   gcagtatatc ctgacagaaa gataatagta ttcctgtttg cagaagacag cggaacagga 3420
   gcgtatgcaa taactaaaga cggagtattt gctacgatag tagcgaaagt aaaagaagga 3480
   gcacctaacg gactcagtgt aatcaaattt gtagaagtag gcggatttgc gaacaatgac 3540
   cttgtagaac agaagacaca gttctttgac ggtggagtaa atgttggaga tacaacagaa 3600
   cctgcaacac ctacaacacc tgtaacaaca ccgacaacaa cagatgatct ggatgcagta 3660
   aggattaaag tggacacagt aaatgcaaaa ccgggagaca cagtaagaat acctgtaaga 3720
   ttcagcggta taccatccaa gggaatagca aactgtgact ttgtatacag ctatgacccg 3780
   aatgtacttg agataataga gatagaaccg ggagaattga tagttgaccc gaatcctacc 3840
   aagagctttg atactgcagt atatcctgac agaaagatga tagtattcct gtttgcggaa 3900
   gacagcggaa caggagcgta tgcaataact gaagatggag tatttgctac gatagtagcg 3960
   aaagtaaaat ccggagcacc taacggactc agtgtaatca aatttgtaga agtaggcgga 4020
   tttgcgaaca atgaccttgt agaacagaag acacagttct ttgacggtgg agtaaatgtt 4080
   ggagatacaa cagaacctgc aacacctaca acacctgtaa caacaccgac aacaacagat 4140
   gatctggatg cagtaaggat taaagtggac acagtaaatg caaaaccggg agacacagta 4200
   agaatacctg taagattcag cggtatacca tccaagggaa tagcaaactg tgactttgta 4260
   tacagctatg acccgaatgt acttgagata atagagatag aaccgggaga cataatagtt 4320
   gacccgaatc ctgacaagag ctttgatact gcagtatatc ctgacagaaa gataatagta 4380
   ttcctgtttg cagaagacag cggaacggga gcgtatgcaa taactaaaga cggagtattt 4440
   gctacgatag tagcgaaagt aaaagaagga gcacctaacg gactcagtgt aatcaaattt 4500
   gtagaagtag gcggatttgc gaacaatgac cttgtagaac agaagacaca gttctttgac 4560
   ggtggagtaa atgttggaga tacaacagta cctacaacat cgccgacaac aacaccgcca 4620
   gagccgacga taactccgaa caagttgaca cttaagatag gcagagcaga aggaagacct 4680
   ggagacacgg tggaaatacc ggttaacttg tatggagtac ctcaaaaagg aatagcaagc 4740
   ggtgacttcg tagtaagcta tgacccgaat gtacttgaga taatagagat agaaccggga 4800
   gaattgatag ttgacccgaa tcctaccaag agctttgata ctgctgcagt atatcctgac 4860
   agaaagatga tagtattcct gtttgcggaa gacagcggaa caggagcgta tgcaataact 4920
   gaagatggag tatttgctac gatagtagcg aaagtaaaag aaggagcacc tgaaggattc 4980
   agtgcaatag aaatttctga gtttggtgca tttgcagata atgatctggt agaagtggaa 5040
   actgacctta tcaatggtgg agtacttgta actaataaac ctgtaataga aggatataaa 5100
   gtatccggat acattttgcc agacttctcc ttcgacgcta ctgttgcacc acttgtaaag 5160
   gccggattca aagttgaaat agtaggaaca gaattgtatg cagtaacaga tgcaaacgga 5220
   tactttgaaa taaccggagt acctgcaaat gcaagcggat atacattgaa gatttcaaga 5280
   gcaacttact tggacagagt aattgcaaat gttgtagtaa cgggagatac ttcagtttca 5340
   acttcacagg ctccaataat gatgtgggta ggagacatag tgaaagacaa ttctatcaac 5400
   ctgttggacg ttgcagaagt tatccgttgc ttcaacgcta ctaaaggaag cgcaaactac 5460
   gtagaagaac ttgacattaa tagaaacggc gcaattaaca tgcaagacat aatgattgtt 5520
   cataagcact ttggagctac atcaagtgat tacgacgcac agtaaatatt aaa 5573

<210> 10
<211> 1854
<212> PRT
<213> Clostridium thermocellum

<400> 10

Met Arg Lys Val Ile Ser Met Leu Leu Val Val Ala Met Leu Thr Thr
1               5                   10                  15

Ile Phe Ala Ala Met Ile Pro Gln Thr Val Ser Ala Ala Thr Met Thr
            20                  25                  30

Val Glu Ile Gly Lys Val Thr Ala Ala Val Gly Ser Lys Val Glu Ile
        35                  40                  45

Pro Ile Thr Leu Lys Gly Val Pro Ser Lys Gly Met Ala Asn Cys Asp
    50                  55                  60

Phe Val Leu Gly Tyr Asp Pro Asn Val Leu Glu Val Thr Glu Val Lys
65                  70                  75                  80

Pro Gly Ser Ile Ile Lys Asp Pro Asp Pro Ser Lys Ser Phe Asp Ser
                85                  90                  95

Ala Ile Tyr Pro Asp Arg Lys Met Ile Val Phe Leu Phe Ala Glu Asp
            100                 105                 110

Ser Gly Arg Gly Thr Tyr Ala Ile Thr Gln Asp Gly Val Phe Ala Thr
        115                 120                 125

Ile Val Ala Thr Val Lys Ser Ala Ala Ala Ala Pro Ile Thr Leu Leu
    130                 135                 140

Glu Val Gly Ala Phe Ala Asp Asn Asp Leu Val Glu Ile Ser Thr Thr
145                 150                 155                 160

Phe Val Ala Gly Gly Val Asn Leu Gly Ser Ser Val Pro Thr Thr Gln
                165                 170                 175

Pro Asn Val Pro Ser Asp Gly Val Val Val Glu Ile Gly Lys Val Thr
            180                 185                 190

Gly Ser Val Gly Thr Thr Val Glu Ile Pro Val Tyr Phe Arg Gly Val
        195                 200                 205

Pro Ser Lys Gly Ile Ala Asn Cys Asp Phe Val Phe Arg Tyr Asp Pro
    210                 215                 220

Asn Val Leu Glu Ile Ile Gly Ile Asp Pro Gly Asp Ile Ile Val Asp
225                 230                 235                 240

Pro Asn Pro Thr Lys Ser Phe Asp Thr Ala Ile Tyr Pro Asp Arg Lys
                245                 250                 255

Ile Ile Val Phe Leu Phe Ala Glu Asp Ser Gly Thr Gly Ala Tyr Ala
            260                 265                 270

Ile Thr Lys Asp Gly Val Phe Ala Lys Ile Arg Ala Thr Val Lys Ser
        275                 280                 285

Ser Ala Pro Gly Tyr Ile Thr Phe Asp Glu Val Gly Gly Phe Ala Asp
    290                 295                 300

Asn Asp Leu Val Glu Gln Lys Val Ser Phe Ile Asp Gly Gly Val Asn
305                 310                 315                 320

Val Gly Asn Ala Thr Pro Thr Lys Gly Ala Thr Pro Thr Asn Thr Ala
                325                 330                 335

Thr Pro Thr Lys Ser Ala Thr Ala Thr Pro Thr Arg Pro Ser Val Pro
            340                 345                 350

Thr Asn Thr Pro Thr Asn Thr Pro Ala Asn Thr Pro Val Ser Gly Asn
        355                 360                 365

Leu Lys Val Glu Phe Tyr Asn Ser Asn Pro Ser Asp Thr Thr Asn Ser
    370                 375                 380

Ile Asn Pro Gln Phe Lys Val Thr Asn Thr Gly Ser Ser Ala Ile Asp
385                 390                 395                 400

Leu Ser Lys Leu Thr Leu Arg Tyr Tyr Tyr Thr Val Asp Gly Gln Lys
                405                 410                 415

Asp Gln Thr Phe Trp Cys Asp His Ala Ala Ile Ile Gly Ser Asn Gly
            420                 425                 430

Ser Tyr Asn Gly Ile Thr Ser Asn Val Lys Gly Thr Phe Val Lys Met
        435                 440                 445

Ser Ser Ser Thr Asn Asn Ala Asp Thr Tyr Leu Glu Ile Ser Phe Thr
    450                 455                 460

Gly Gly Thr Leu Glu Pro Gly Ala His Val Gln Ile Gln Gly Arg Phe
465                 470                 475                 480

Ala Lys Asn Asp Trp Ser Asn Tyr Thr Gln Ser Asn Asp Tyr Ser Phe
                485                 490                 495

Lys Ser Ala Ser Gln Phe Val Glu Trp Asp Gln Val Thr Ala Tyr Leu
            500                 505                 510

Asn Gly Val Leu Val Trp Gly Lys Glu Pro Gly Gly Ser Val Val Pro
        515                 520                 525

Ser Thr Gln Pro Val Thr Thr Pro Pro Ala Thr Thr Lys Pro Pro Ala
    530                 535                 540

Thr Thr Lys Pro Pro Ala Thr Thr Ile Pro Pro Ser Asp Asp Pro Asn
545                 550                 555                 560

Ala Ile Lys Ile Lys Val Asp Thr Val Asn Ala Lys Pro Gly Asp Thr
                565                 570                 575

Val Asn Ile Pro Val Arg Phe Ser Gly Ile Pro Ser Lys Gly Ile Ala
            580                 585                 590

Asn Cys Asp Phe Val Tyr Ser Tyr Asp Pro Asn Val Leu Glu Ile Ile
        595                 600                 605

Glu Ile Lys Pro Gly Glu Leu Ile Val Asp Pro Asn Pro Asp Lys Ser
    610                 615                 620

Phe Asp Thr Ala Val Tyr Pro Asp Arg Lys Ile Ile Val Phe Leu Phe

625                 630                 635                 640

Ala Glu Asp Ser Gly Thr Gly Ala Tyr Ala Ile Thr Lys Asp Gly Val
                645                 650                 655

Phe Ala Thr Ile Val Ala Lys Val Lys Ser Gly Ala Pro Asn Gly Leu
            660                 665                 670

Ser Val Ile Lys Phe Val Glu Val Gly Gly Phe Ala Asn Asn Asp Leu
        675                 680                 685

Val Glu Gln Arg Thr Gln Phe Phe Asp Gly Gly Val Asn Val Gly Asp
    690                 695                 700

Thr Thr Val Pro Thr Thr Pro Thr Thr Pro Val Thr Thr Pro Thr Asp
705                 710                 715                 720

Asp Ser Asn Ala Val Arg Ile Lys Val Asp Thr Val Asn Ala Lys Pro
                725                 730                 735

Gly Asp Thr Val Arg Ile Pro Val Arg Phe Ser Gly Ile Pro Ser Lys
            740                 745                 750

Gly Ile Ala Asn Cys Asp Phe Val Tyr Ser Tyr Asp Pro Asn Val Leu
        755                 760                 765

Glu Ile Ile Glu Ile Glu Pro Gly Asp Ile Ile Val Asp Pro Asn Pro
    770                 775                 780

Asp Lys Ser Phe Asp Thr Ala Val Tyr Pro Asp Arg Lys Ile Ile Val
785                 790                 795                 800

Phe Leu Phe Ala Glu Asp Ser Gly Thr Gly Ala Tyr Ala Ile Thr Lys
                805                 810                 815

Asp Gly Val Phe Ala Thr Ile Val Ala Lys Val Lys Ser Gly Ala Pro
            820                 825                 830

Asn Gly Leu Ser Val Ile Lys Phe Val Glu Val Gly Gly Phe Ala Asn
        835                 840                 845

Asn Asp Leu Val Glu Gln Lys Thr Gln Phe Phe Asp Gly Gly Val Asn
    850                 855                 860

Val Gly Asp Thr Thr Glu Pro Ala Thr Pro Thr Thr Pro Val Thr Thr
865                 870                 875                 880

Pro Thr Thr Thr Asp Asp Leu Asp Ala Val Arg Ile Lys Val Asp Thr
                885                 890                 895

Val Asn Ala Lys Pro Gly Asp Thr Val Arg Ile Pro Val Arg Phe Ser
            900                 905                 910

Gly Ile Pro Ser Lys Gly Ile Ala Asn Cys Asp Phe Val Tyr Ser Tyr
        915                 920                 925

Asp Pro Asn Val Leu Glu Ile Ile Glu Ile Glu Pro Gly Asp Ile Ile
    930                 935                 940

Val Asp Pro Asn Pro Asp Lys Ser Phe Asp Thr Ala Val Tyr Pro Asp
945                 950                 955                 960

Arg Lys Ile Ile Val Phe Leu Phe Ala Glu Asp Ser Gly Thr Gly Ala
                965                 970                 975

Tyr Ala Ile Thr Lys Asp Gly Val Phe Ala Thr Ile Val Ala Lys Val
            980                 985                 990

Lys Ser Gly Ala Pro Asn Gly Leu  Ser Val Ile Lys Phe  Val Glu Val
        995                 1000                1005

Gly Gly  Phe Ala Asn Asn Asp  Leu Val Glu Gln Lys  Thr Gln Phe
    1010                1015                1020

Phe Asp  Gly Gly Val Asn Val  Gly Asp Thr Thr Glu  Pro Ala Thr
    1025                1030                1035

Pro Thr  Thr Pro Val Thr Thr  Pro Thr Thr Thr Asp  Asp Leu Asp
    1040                1045                1050

Ala Val  Arg Ile Lys Val Asp  Thr Val Asn Ala Lys  Pro Gly Asp
    1055                1060                1065

Thr Val  Arg Ile Pro Val Arg  Phe Ser Gly Ile Pro  Ser Lys Gly
    1070                1075                1080

Ile Ala  Asn Cys Asp Phe Val  Tyr Ser Tyr Asp Pro  Asn Val Leu
    1085                1090                1095

Glu Ile  Ile Glu Ile Glu Pro  Gly Asp Ile Ile Val  Asp Pro Asn
    1100                1105                1110

Pro Asp  Lys Ser Phe Asp Thr  Ala Val Tyr Pro Asp  Arg Lys Ile
    1115                1120                1125

Ile Val Phe Leu Phe Ala Glu Asp Ser Gly Thr Gly Ala Tyr Ala
    1130                1135                1140

Ile Thr Lys Asp Gly Val Phe Ala Thr Ile Val Ala Lys Val Lys
    1145                1150                1155

Glu Gly Ala Pro Asn Gly Leu Ser Val Ile Lys Phe Val Glu Val
    1160                1165                1170

Gly Gly Phe Ala Asn Asn Asp Leu Val Glu Gln Lys Thr Gln Phe
    1175                1180                1185

Phe Asp Gly Gly Val Asn Val Gly Asp Thr Thr Glu Pro Ala Thr
    1190                1195                1200

Pro Thr Thr Pro Val Thr Thr Pro Thr Thr Thr Asp Asp Leu Asp
    1205                1210                1215

Ala Val Arg Ile Lys Val Asp Thr Val Asn Ala Lys Pro Gly Asp
    1220                1225                1230

Thr Val Arg Ile Pro Val Arg Phe Ser Gly Ile Pro Ser Lys Gly
    1235                1240                1245

Ile Ala Asn Cys Asp Phe Val Tyr Ser Tyr Asp Pro Asn Val Leu
    1250                1255                1260

Glu Ile Ile Glu Ile Glu Pro Gly Glu Leu Ile Val Asp Pro Asn
    1265                1270                1275

Pro Thr Lys Ser Phe Asp Thr Ala Val Tyr Pro Asp Arg Lys Met
    1280                1285                1290

Ile Val Phe Leu Phe Ala Glu Asp Ser Gly Thr Gly Ala Tyr Ala
    1295                1300                1305

Ile Thr Glu Asp Gly Val Phe Ala Thr Ile Val Ala Lys Val Lys
    1310                1315                1320

Ser Gly Ala Pro Asn Gly Leu Ser Val Ile Lys Phe Val Glu Val
    1325                1330                1335

Gly Gly Phe Ala Asn Asn Asp Leu Val Glu Gln Lys Thr Gln Phe
    1340                1345                1350

Phe Asp Gly Gly Val Asn Val Gly Asp Thr Thr Glu Pro Ala Thr
    1355                1360                1365

Pro Thr Thr Pro Val Thr Thr Pro Thr Thr Thr Asp Asp Leu Asp
    1370                1375                1380

Ala Val Arg Ile Lys Val Asp Thr Val Asn Ala Lys Pro Gly Asp
    1385                1390                1395

Thr Val Arg Ile Pro Val Arg Phe Ser Gly Ile Pro Ser Lys Gly
    1400                1405                1410

Ile Ala Asn Cys Asp Phe Val Tyr Ser Tyr Asp Pro Asn Val Leu
    1415                1420                1425

Glu Ile Ile Glu Ile Glu Pro Gly Asp Ile Ile Val Asp Pro Asn
    1430                1435                1440

Pro Asp Lys Ser Phe Asp Thr Ala Val Tyr Pro Asp Arg Lys Ile
    1445                1450                1455

Ile Val Phe Leu Phe Ala Glu Asp Ser Gly Thr Gly Ala Tyr Ala
    1460                1465                1470

Ile Thr Lys Asp Gly Val Phe Ala Thr Ile Val Ala Lys Val Lys
    1475                1480                1485

Glu Gly Ala Pro Asn Gly Leu Ser Val Ile Lys Phe Val Glu Val
    1490                1495                1500

Gly Gly Phe Ala Asn Asn Asp Leu Val Glu Gln Lys Thr Gln Phe
    1505                1510                1515

Phe Asp Gly Gly Val Asn Val Gly Asp Thr Thr Val Pro Thr Thr
    1520                1525                1530

Ser Pro Thr Thr Thr Pro Pro Glu Pro Thr Ile Thr Pro Asn Lys
    1535                1540                1545

Leu Thr Leu Lys Ile Gly Arg Ala Glu Gly Arg Pro Gly Asp Thr
    1550                1555                1560

Val Glu Ile Pro Val Asn Leu Tyr Gly Val Pro Gln Lys Gly Ile
    1565                1570                1575

Ala Ser Gly Asp Phe Val Val Ser Tyr Asp Pro Asn Val Leu Glu
    1580                1585                1590

Ile Ile Glu Ile Glu Pro Gly Glu Leu Ile Val Asp Pro Asn Pro

1595                1600                1605

Thr Lys Ser Phe Asp Thr Ala Ala Val Tyr Pro Asp Arg Lys Met
    1610                1615                1620

Ile Val Phe Leu Phe Ala Glu Asp Ser Gly Thr Gly Ala Tyr Ala
    1625                1630                1635

Ile Thr Glu Asp Gly Val Phe Ala Thr Ile Val Ala Lys Val Lys
    1640                1645                1650

Glu Gly Ala Pro Glu Gly Phe Ser Ala Ile Glu Ile Ser Glu Phe
    1655                1660                1665

Gly Ala Phe Ala Asp Asn Asp Leu Val Glu Val Glu Thr Asp Leu
    1670                1675                1680

Ile Asn Gly Gly Val Leu Val Thr Asn Lys Pro Val Ile Glu Gly
    1685                1690                1695

Tyr Lys Val Ser Gly Tyr Ile Leu Pro Asp Phe Ser Phe Asp Ala
    1700                1705                1710

Thr Val Ala Pro Leu Val Lys Ala Gly Phe Lys Val Glu Ile Val
    1715                1720                1725

Gly Thr Glu Leu Tyr Ala Val Thr Asp Ala Asn Gly Tyr Phe Glu
    1730                1735                1740

Ile Thr Gly Val Pro Ala Asn Ala Ser Gly Tyr Thr Leu Lys Ile
    1745                1750                1755

Ser Arg Ala Thr Tyr Leu Asp Arg Val Ile Ala Asn Val Val Val
    1760                1765                1770

Thr Gly Asp Thr Ser Val Ser Thr Ser Gln Ala Pro Ile Met Met
    1775                1780                1785

Trp Val Gly Asp Ile Val Lys Asp Asn Ser Ile Asn Leu Leu Asp
    1790                1795                1800

Val Ala Glu Val Ile Arg Cys Phe Asn Ala Thr Lys Gly Ser Ala
    1805                1810                1815

Asn Tyr Val Glu Glu Leu Asp Ile Asn Arg Asn Gly Ala Ile Asn
    1820                1825                1830

Met Gln Asp Ile Met Ile Val His Lys His Phe Gly Ala Thr Ser
    1835                1840                1845

Ser Asp Tyr Asp Ala Gln
    1850

<210> 11
<211> 1599
<212> DNA
<213> Clostridium thermocellum

<400> 11
atgagaaaag tcatcagtat gctcttagtt gtggctatgc tgacgacgat ttttgcggcg      60

atgataccgc agacagtatc ggcggccaca atgacagtcg agatcggcaa agttacagca     120

gccgttggat caaaagtaga aatacctata accctgaaag gagtgccatc caaaggaatg     180

gccaattgcg acttcgtatt gggttatgat ccaaatgtgc tggaagtaac agaagtaaaa     240

ccaggaagca taataaaaga tccggatcct agcaagagct ttgatagcgc aatatatccg     300

gatcgaaaga tgattgtatt tctgtttgca gaagacagtg gaagaggaac gtatgcaata     360

actcaggatg gagtatttgc aacaattgta gccactgtca aatcagctgc agcggcaccg     420

attactttgc ttgaagtagg tgcatttgcg gacaacgatt tagtagaaat aagcacaact     480

tttgtcgcgg gcggagtaaa tcttggtagt tccgtaccga caacacagcc aaatgttccg     540

tcagacggtg tggtagtaga aattggcaaa gttacgggat ctgttggaac tacagttgaa     600

atacctgtat atttcagagg agttccatcc aaaggaatag caaactgcga ctttgtgttc     660

agatatgatc cgaatgtatt ggaaattata gggatagatc ccggagacat aatagttgac     720

ccgaatccta ccaagagctt tgatactgca atatatcctg acagaaagat aatagtattc     780

ctgtttgcgg aagacagcgg aacaggagcg tatgcaataa ctaaagacgg agtatttgca     840

aaaataagag caactgtaaa atcaagtgct ccgggctata ttactttcga cgaagtaggt     900

ggatttgcag ataatgacct ggtagaacag aaggtatcat ttatagacgg tggtgttaac     960

gttggcaatg caacaccgac caagggagca acaccaacaa atacagctac gccgacaaaa    1020

tcagctacgg ctacgcccac caggccatcg gtaccgacaa acacaccgac aaacacaccg    1080

gcaaatacac cggtatcagg caatttgaag gttgaattct acaacagcaa tccttcagat    1140

actactaact caatcaatcc tcagttcaag gttactaata ccggaagcag tgcaattgat    1200

ttgtccaaac tcacattgag atattattat acagtagacg gacagaaaga tcagaccttc    1260

tggtgtgacc atgctgcaat aatcggcagt aacggcagct acaacggaat tacttcaaat    1320

gtaaaaggaa catttgtaaa aatgagttcc tcaacaaata acgcagacac ctaccttgaa    1380

ataagcttta caggcggaac tcttgaaccg ggtgcacatg ttcagataca aggtagattt    1440

gcaaagaatg actggagtaa ctatacacag tcaaatgact actcattcaa gtctgcttca    1500

cagtttgttg aatgggatca ggtaacagca tacttgaacg gtgttcttgt atggggtaaa    1560

gaacccggtg gcagtgtagt accatcaaca cagcctgta                           1599

<210>  12
<211>  533
<212>  PRT
<213>  Clostridium thermocellum

<400>  12

Met Arg Lys Val Ile Ser Met Leu Leu Val Val Ala Met Leu Thr Thr
1               5                   10                  15

Ile Phe Ala Ala Met Ile Pro Gln Thr Val Ser Ala Ala Thr Met Thr
            20                  25                  30

Val Glu Ile Gly Lys Val Thr Ala Ala Val Gly Ser Lys Val Glu Ile
        35                  40                  45

Pro Ile Thr Leu Lys Gly Val Pro Ser Lys Gly Met Ala Asn Cys Asp
    50                  55                  60

Phe Val Leu Gly Tyr Asp Pro Asn Val Leu Glu Val Thr Glu Val Lys
65                  70                  75                  80

Pro Gly Ser Ile Ile Lys Asp Pro Asp Pro Ser Lys Ser Phe Asp Ser
                85                  90                  95

Ala Ile Tyr Pro Asp Arg Lys Met Ile Val Phe Leu Phe Ala Glu Asp
            100                 105                 110

Ser Gly Arg Gly Thr Tyr Ala Ile Thr Gln Asp Gly Val Phe Ala Thr
        115                 120                 125

Ile Val Ala Thr Val Lys Ser Ala Ala Ala Ala Pro Ile Thr Leu Leu
    130                 135                 140

Glu Val Gly Ala Phe Ala Asp Asn Asp Leu Val Glu Ile Ser Thr Thr
145                 150                 155                 160

Phe Val Ala Gly Gly Val Asn Leu Gly Ser Ser Val Pro Thr Thr Gln
                165                 170                 175

Pro Asn Val Pro Ser Asp Gly Val Val Val Glu Ile Gly Lys Val Thr
            180                 185                 190

Gly Ser Val Gly Thr Thr Val Glu Ile Pro Val Tyr Phe Arg Gly Val
        195                 200                 205

Pro Ser Lys Gly Ile Ala Asn Cys Asp Phe Val Phe Arg Tyr Asp Pro
    210                 215                 220

Asn Val Leu Glu Ile Ile Gly Ile Asp Pro Gly Asp Ile Ile Val Asp
225                 230                 235                 240

Pro Asn Pro Thr Lys Ser Phe Asp Thr Ala Ile Tyr Pro Asp Arg Lys
                245                 250                 255

Ile Ile Val Phe Leu Phe Ala Glu Asp Ser Gly Thr Gly Ala Tyr Ala
            260                 265                 270

Ile Thr Lys Asp Gly Val Phe Ala Lys Ile Arg Ala Thr Val Lys Ser
        275                 280                 285

Ser Ala Pro Gly Tyr Ile Thr Phe Asp Glu Val Gly Gly Phe Ala Asp
    290                 295                 300

Asn Asp Leu Val Glu Gln Lys Val Ser Phe Ile Asp Gly Gly Val Asn
305                 310                 315                 320

Val Gly Asn Ala Thr Pro Thr Lys Gly Ala Thr Pro Thr Asn Thr Ala
                325                 330                 335

Thr Pro Thr Lys Ser Ala Thr Ala Thr Pro Thr Arg Pro Ser Val Pro
            340                 345                 350

Thr Asn Thr Pro Thr Asn Thr Pro Ala Asn Thr Pro Val Ser Gly Asn
        355                 360                 365

Leu Lys Val Glu Phe Tyr Asn Ser Asn Pro Ser Asp Thr Thr Asn Ser
    370                 375                 380

Ile Asn Pro Gln Phe Lys Val Thr Asn Thr Gly Ser Ser Ala Ile Asp
385                 390                 395                 400

Leu Ser Lys Leu Thr Leu Arg Tyr Tyr Tyr Thr Val Asp Gly Gln Lys
                405                 410                 415

Asp Gln Thr Phe Trp Cys Asp His Ala Ala Ile Ile Gly Ser Asn Gly
            420                 425                 430

Ser Tyr Asn Gly Ile Thr Ser Asn Val Lys Gly Thr Phe Val Lys Met
        435                 440                 445

Ser Ser Ser Thr Asn Asn Ala Asp Thr Tyr Leu Glu Ile Ser Phe Thr

450                 455                 460

Gly Gly Thr Leu Glu Pro Gly Ala His Val Gln Ile Gln Gly Arg Phe
465                 470                 475                 480

Ala Lys Asn Asp Trp Ser Asn Tyr Thr Gln Ser Asn Asp Tyr Ser Phe
                485                 490                 495

Lys Ser Ala Ser Gln Phe Val Glu Trp Asp Gln Val Thr Ala Tyr Leu
            500                 505                 510

Asn Gly Val Leu Val Trp Gly Lys Glu Pro Gly Gly Ser Val Val Pro
        515                 520                 525

Ser Thr Gln Pro Val
    530

<210>  13
<211>  408
<212>  DNA
<213>  Clostridium thermocellum

<400>  13
atgacagtcg agatcggcaa agttacagca gccgttggat caaaagtaga aatacctata      60

accctgaaag gagtgccatc caaaggaatg gccaattgcg acttcgtatt gggttatgat     120

ccaaatgtgc tggaagtaac agaagtaaaa ccaggaagca taataaaaga tccggatcct     180

agcaagagct ttgatagcgc aatatatccg gatcgaaaga tgattgtatt tctgtttgca     240

gaagacagtg gaagaggaac gtatgcaata actcaggatg gagtatttgc aacaattgta     300

gccactgtca aatcagctgc agcggcaccg attactttgc ttgaagtagg tgcatttgcg     360

gacaacgatt tagtagaaat aagcacaact tttgtcgcgg gcggagta                  408

<210>  14
<211>  136
<212>  PRT
<213>  Clostridium thermocellum

<400>  14

Met Thr Val Glu Ile Gly Lys Val Thr Ala Ala Val Gly Ser Lys Val
1               5                   10                  15

Glu Ile Pro Ile Thr Leu Lys Gly Val Pro Ser Lys Gly Met Ala Asn
            20                  25                  30

Cys Asp Phe Val Leu Gly Tyr Asp Pro Asn Val Leu Glu Val Thr Glu
        35                  40                  45

Val Lys Pro Gly Ser Ile Ile Lys Asp Pro Asp Pro Ser Lys Ser Phe
    50                  55                  60

Asp Ser Ala Ile Tyr Pro Asp Arg Lys Met Ile Val Phe Leu Phe Ala
65                  70                  75                  80

Glu Asp Ser Gly Arg Gly Thr Tyr Ala Ile Thr Gln Asp Gly Val Phe
                85                  90                  95

Ala Thr Ile Val Ala Thr Val Lys Ser Ala Ala Ala Ala Pro Ile Thr
            100                 105                 110

Leu Leu Glu Val Gly Ala Phe Ala Asp Asn Asp Leu Val Glu Ile Ser
        115                 120                 125

Thr Thr Phe Val Ala Gly Gly Val
    130                 135

<210>  15
<211>  237
<212>  PRT
<213>  Clostridium thermocellum

<400>  15

Ala Thr Pro Thr Lys Gly Ala Thr Pro Thr Asn Thr Ala Thr Pro Thr
1               5                   10                  15

Lys Ser Ala Thr Ala Thr Pro Thr Arg Pro Ser Val Pro Thr Asn Thr
            20                  25                  30

Pro Thr Asn Thr Pro Ala Asn Thr Pro Val Ser Gly Asn Leu Lys Val
        35                  40                  45

Glu Phe Tyr Asn Ser Asn Pro Ser Asp Thr Thr Asn Ser Ile Asn Pro
    50                  55                  60

Gln Phe Lys Val Thr Asn Thr Gly Ser Ser Ala Ile Asp Leu Ser Lys
65                  70                  75                  80

Leu Thr Leu Arg Tyr Tyr Tyr Thr Val Asp Gly Gln Lys Asp Gln Thr
                85                  90                  95

Phe Trp Cys Asp His Ala Ala Ile Ile Gly Ser Asn Gly Ser Tyr Asn
            100                 105                 110

Gly Ile Thr Ser Asn Val Lys Gly Thr Phe Val Lys Met Ser Ser Ser
        115                 120                 125

Thr Asn Asn Ala Asp Thr Tyr Leu Glu Ile Ser Phe Thr Gly Gly Thr
    130                 135                 140

Leu Glu Pro Gly Ala His Val Gln Ile Gln Gly Arg Phe Ala Lys Asn
145                 150                 155                 160

Asp Trp Ser Asn Tyr Thr Gln Ser Asn Asp Tyr Ser Phe Lys Ser Ala
                165                 170                 175

Ser Gln Phe Val Glu Trp Asp Gln Val Thr Ala Tyr Leu Asn Gly Val
            180                 185                 190

Leu Val Trp Gly Lys Glu Pro Gly Gly Ser Val Val Pro Ser Thr Gln
        195                 200                 205

Pro Val Thr Thr Pro Pro Ala Thr Thr Lys Pro Pro Ala Thr Thr Lys
    210                 215                 220

Pro Pro Ala Thr Thr Ile Pro Pro Ser Asp Asp Pro Asn
225                 230                 235

<210>  16
<211>  780
<212>  DNA
<213>  Clostridium thermocellum

<400>  16
ggatttgcag ataatgacct ggtagaacag aaggtatcat ttatagacgg tggtgttaac      60

gttggcaatg caacaccgac caagggagca acaccaacaa atacagctac gccgacaaaa     120

tcagctacgg ctacgcccac caggccatcg gtaccgacaa acacaccgac aaacacaccg     180

gcaaatacac cggtatcagg caatttgaag gttgaattct acaacagcaa tccttcagat     240

actactaact caatcaatcc tcagttcaag gttactaata ccggaagcag tgcaattgat     300

ttgtccaaac tcacattgag atattattat acagtagacg gacagaaaga tcagaccttc     360

tggtgtgacc atgctgcaat aatcggcagt aacggcagct acaacggaat tacttcaaat     420

gtaaaaggaa catttgtaaa aatgagttcc tcaacaaata acgcagacac ctaccttgaa     480

ataagcttta caggcggaac tcttgaaccg ggtgcacatg ttcagataca aggtagattt     540

gcaaagaatg actggagtaa ctatacacag tcaaatgact actcattcaa gtctgcttca     600

cagtttgttg aatgggatca ggtaacagca tacttgaacg gtgttcttgt atggggtaaa     660

gaacccggtg gcagtgtagt accatcaaca cagcctgtaa caacaccacc tgcaacaaca     720

aaaccacctg caacaacaaa accacctgca acaacaatac cgccgtcaga tgatccgaat     780

<210>  17
<211>  1434

<212>  DNA
<213>  Clostridium thermocellum

<400>  17
gtgaagaacg taaaaaaaag agtaggtgtg gttttgctga ttcttgcagt gttgggggtt      60

tatatgttgg caatgccggc aaacactgtg tcagcggcag gtgtgccttt taacacaaaa     120

tacccctatg gtcctacttc tattgccgat aatcagtcgg aagtaactgc aatgctcaaa     180

gcagaatggg aagactggaa gagcaagaga attacctcga acggtgcagg aggatacaag     240

agagtacagc gtgatgcttc caccaattat gatacggtat ccgaaggtat gggatacgga     300

cttcttttgg cggtttgctt taacgaacag gctttgtttg acgatttata ccgttacgta     360

aaatctcatt tcaatggaaa cggacttatg cactggcaca ttgatgccaa caacaatgtt     420

acaagtcatg acggcggcga cggtgcggca accgatgctg atgaggatat tgcacttgcg     480

ctcatatttg cggacaagtt atggggttct tccggtgcaa taaactacgg gcaggaagca     540

aggacattga taaacaatct ttacaaccat tgtgtagagc atggatccta tgtattaaag     600

cccggtgaca gatggggagg ttcatcagta acaaacccgt catattttgc gcctgcatgg     660

tacaaagtgt atgctcaata tacaggagac acaagatgga atcaagtggc ggacaagtgt     720

taccaaattg ttgaagaagt taagaaatac aacaacggaa ccggccttgt tcctgactgg     780

tgtactgcaa gcggaactcc ggcaagcggt cagagttacg actacaaata tgatgctaca     840

cgttacggct ggagaactgc cgtggactat tcatggtttg gtgaccagag agcaaaggca     900

aactgcgata tgctgaccaa attctttgcc agagacgggg caaaaggaat cgttgacgga     960

tacacaattc aaggttcaaa aattagcaac aatcacaacg catcatttat aggacctgtt    1020

gcggcagcaa gtatgacagg ttacgatttg aactttgcaa aggaacttta tagggagact    1080

gttgctgtaa aggacagtga atattacgga tattacggaa acagcttgag actgctcact    1140

ttgttgtaca taacaggaaa cttcccgaat cctttgagtg acctttccgg ccaaccgaca    1200

ccaccgtcga atccgacacc ttcattgcct cctcaggttg tttacggtga tgtaaatggc    1260

gacggtaatg ttaactccac tgatttgact atgttaaaaa gatatctgct gaagagtgtt    1320

accaatataa acagagaggc tgcagacgtt aatcgtgacg gtgcgattaa ctcctctgac    1380

atgactatat taaagagata tctgataaag agcatacccc acctacctta ttag          1434


<210>  18
<211>  477
<212>  PRT
<213>  Clostridium thermocellum

<400>  18

Met Lys Asn Val Lys Lys Arg Val Gly Val Val Leu Leu Ile Leu Ala
1               5                   10                  15

Val Leu Gly Val Tyr Met Leu Ala Met Pro Ala Asn Thr Val Ser Ala
            20                  25                  30

Ala Gly Val Pro Phe Asn Thr Lys Tyr Pro Tyr Gly Pro Thr Ser Ile
        35                  40                  45

Ala Asp Asn Gln Ser Glu Val Thr Ala Met Leu Lys Ala Glu Trp Glu
    50                  55                  60

Asp Trp Lys Ser Lys Arg Ile Thr Ser Asn Gly Ala Gly Gly Tyr Lys
65                  70                  75                  80

Arg Val Gln Arg Asp Ala Ser Thr Asn Tyr Asp Thr Val Ser Glu Gly
                85                  90                  95

Met Gly Tyr Gly Leu Leu Leu Ala Val Cys Phe Asn Glu Gln Ala Leu
            100                 105                 110

Phe Asp Asp Leu Tyr Arg Tyr Val Lys Ser His Phe Asn Gly Asn Gly
        115                 120                 125

Leu Met His Trp His Ile Asp Ala Asn Asn Asn Val Thr Ser His Asp
    130                 135                 140

Gly Gly Asp Gly Ala Ala Thr Asp Ala Asp Glu Asp Ile Ala Leu Ala
145                 150                 155                 160

Leu Ile Phe Ala Asp Lys Leu Trp Gly Ser Ser Gly Ala Ile Asn Tyr
                165                 170                 175

Gly Gln Glu Ala Arg Thr Leu Ile Asn Asn Leu Tyr Asn His Cys Val
            180                 185                 190

Glu His Gly Ser Tyr Val Leu Lys Pro Gly Asp Arg Trp Gly Gly Ser
        195                 200                 205

Ser Val Thr Asn Pro Ser Tyr Phe Ala Pro Ala Trp Tyr Lys Val Tyr
    210                 215                 220

Ala Gln Tyr Thr Gly Asp Thr Arg Trp Asn Gln Val Ala Asp Lys Cys
225                 230                 235                 240

Tyr Gln Ile Val Glu Glu Val Lys Lys Tyr Asn Asn Gly Thr Gly Leu
                245                 250                 255

Val Pro Asp Trp Cys Thr Ala Ser Gly Thr Pro Ala Ser Gly Gln Ser
            260                 265                 270

Tyr Asp Tyr Lys Tyr Asp Ala Thr Arg Tyr Gly Trp Arg Thr Ala Val
        275                 280                 285

Asp Tyr Ser Trp Phe Gly Asp Gln Arg Ala Lys Ala Asn Cys Asp Met
    290                 295                 300

Leu Thr Lys Phe Phe Ala Arg Asp Gly Ala Lys Gly Ile Val Asp Gly
305                 310                 315                 320

Tyr Thr Ile Gln Gly Ser Lys Ile Ser Asn Asn His Asn Ala Ser Phe
                325                 330                 335

Ile Gly Pro Val Ala Ala Ala Ser Met Thr Gly Tyr Asp Leu Asn Phe
            340                 345                 350

Ala Lys Glu Leu Tyr Arg Glu Thr Val Ala Val Lys Asp Ser Glu Tyr
        355                 360                 365

Tyr Gly Tyr Tyr Gly Asn Ser Leu Arg Leu Leu Thr Leu Leu Tyr Ile
    370                 375                 380

Thr Gly Asn Phe Pro Asn Pro Leu Ser Asp Leu Ser Gly Gln Pro Thr
385                 390                 395                 400

Pro Pro Ser Asn Pro Thr Pro Ser Leu Pro Pro Gln Val Val Tyr Gly
                405                 410                 415

Asp Val Asn Gly Asp Gly Asn Val Asn Ser Thr Asp Leu Thr Met Leu
            420                 425                 430

Lys Arg Tyr Leu Leu Lys Ser Val Thr Asn Ile Asn Arg Glu Ala Ala
        435                 440                 445

Asp Val Asn Arg Asp Gly Ala Ile Asn Ser Ser Asp Met Thr Ile Leu
    450                 455                 460

Lys Arg Tyr Leu Ile Lys Ser Ile Pro His Leu Pro Tyr
465                 470                 475

<210>  19
<211>  2226
<212>  DNA
<213>  Clostridium thermocellum

<400>  19
atggtaaaaa gcagaaagat ttctattctg ttggcagttg caatgctggt atccataatg      60

atacccacaa ctgcattcgc aggtcctaca aaggcaccta caaaagatgg gacatcttat     120

aaggatcttt tccttgaact ctacggaaaa attaaagatc ctaagaacgg atatttcagc 180

ccagacgagg gaattcctta tcactcaatt gaaacattga tcgttgaagc gccggactac 240

ggtcacgtta ctaccagtga ggctttcagc tattatgtat ggcttgaagc aatgtatgga 300

aatctcacag gcaactggtc cggagtagaa acagcatgga aagttatgga ggattggata 360

attcctgaca gcacagagca gccgggtatg tcttcttaca atccaaacag ccctgccaca 420

tatgctgacg aatatgagga tccttcatac tatccttcag agttgaagtt tgataccgta 480

agagttggat ccgaccctgt acacaacgac cttgtatccg catacggtcc taacatgtac 540

ctcatgcact ggttgatgga cgttgacaac tggtacggtt ttggtacagg aacacgggca 600

acattcataa acaccttcca aagaggtgaa caggaatcca catgggaaac cattcctcat 660

ccgtcaatag aagagttcaa atacggcgga ccgaacggat tccttgattt gtttacaaag 720

gacagatcat atgcaaaaca gtggcgttat acaaacgctc ctgacgcaga aggccgtgct 780

atacaggctg tttactgggc aaacaaatgg gcaaaggagc agggtaaagg ttctgccgtt 840

gcttccgttg tatccaaggc tgcaaagatg ggtgacttct tgagaaacga catgttcgac 900

aaatacttca tgaagatcgg tgcacaggac aagactcctg ctaccggtta tgacagtgca 960

cactacctta tggcctggta tactgcatgg ggtggtggaa ttggtgcatc ctgggcatgg 1020

aagatcggat gcagccacgc acacttcgga tatcagaacc cattccaggg atgggtaagt 1080

gcaacacaga gcgactttgc tcctaaatca tccaacggta agagagactg gacaacaagc 1140

tacaagagac agcttgaatt ctatcagtgg ttgcagtcgg ctgaaggtgg tattgccggt 1200

ggagcaacca actcctggaa cggtagatat gagaaatatc ctgctggtac gtcaacgttc 1260

tatggtatgg catatgttcc gcatcctgta tacgctgacc cgggtagtaa ccagtggttc 1320

ggattccagg catggtcaat gcagcgtgta atggagtact acctcgaaac aggagattca 1380

tcagttaaga atttgattaa gaagtgggtc gactgggtaa tgagcgaaat taagctctat 1440

gacgatggaa catttgcaat tcctagcgac ctcgagtggt caggtcagcc tgatacatgg 1500

accggaacat acacaggcaa cccgaacctc catgtaagag taacttctta cggtactgac 1560

cttggtgttg caggttcact tgcaaatgct cttgcaactt atgccgcagc tacagaaaga 1620

tgggaaggaa aacttgatac aaaagcaaga gacatggctg ctgaactggt taaccgtgca 1680

tggtacaact tctactgctc tgaaggaaaa ggtgttgtta ctgaggaagc acgtgctgac 1740

tacaaacgtt tctttgagca ggaagtatac gttccggcag gttggagcgg tactatgccg 1800

aacggtgaca agattcagcc tggtattaag ttcatagaca tccgtacaaa atatagacaa 1860

gatccttact acgatatagt atatcaggca tacttgagag gcgaagctcc tgtattgaat 1920

tatcaccgct tctggcatga agttgacctt gcagttgcaa tgggtgtatt ggctacatac 1980

ttcccggata tgacatataa agtacctggt actccttcta ctaaattata cggcgacgtc 2040

aatgatgacg gaaaagttaa ctcaactgac gctgtagcat tgaagagata tgttttgaga 2100

tcaggtataa gcatcaacac tgacaatgcc gatttgaatg aagacggcag agttaattca 2160

actgacttag gaattttgaa gagatatatt ctcaaagaaa tagatacatt gccgtacaag 2220

aactaa 2226

<210> 20
<211> 741
<212> PRT
<213> Clostridium thermocellum

<400> 20

Met Val Lys Ser Arg Lys Ile Ser Ile Leu Leu Ala Val Ala Met Leu
1               5                   10                  15

Val Ser Ile Met Ile Pro Thr Thr Ala Phe Ala Gly Pro Thr Lys Ala
            20                  25                  30

Pro Thr Lys Asp Gly Thr Ser Tyr Lys Asp Leu Phe Leu Glu Leu Tyr
        35                  40                  45

Gly Lys Ile Lys Asp Pro Lys Asn Gly Tyr Phe Ser Pro Asp Glu Gly
    50                  55                  60

Ile Pro Tyr His Ser Ile Glu Thr Leu Ile Val Glu Ala Pro Asp Tyr
65                  70                  75                  80

Gly His Val Thr Thr Ser Glu Ala Phe Ser Tyr Tyr Val Trp Leu Glu
                85                  90                  95

Ala Met Tyr Gly Asn Leu Thr Gly Asn Trp Ser Gly Val Glu Thr Ala
            100                 105                 110

Trp Lys Val Met Glu Asp Trp Ile Ile Pro Asp Ser Thr Glu Gln Pro
        115                 120                 125

Gly Met Ser Ser Tyr Asn Pro Asn Ser Pro Ala Thr Tyr Ala Asp Glu
    130                 135                 140

Tyr Glu Asp Pro Ser Tyr Tyr Pro Ser Glu Leu Lys Phe Asp Thr Val
145                 150                 155                 160

Arg Val Gly Ser Asp Pro Val His Asn Asp Leu Val Ser Ala Tyr Gly
                165                 170                 175

Pro Asn Met Tyr Leu Met His Trp Leu Met Asp Val Asp Asn Trp Tyr

            180                 185                 190

Gly Phe Gly Thr Gly Thr Arg Ala Thr Phe Ile Asn Thr Phe Gln Arg
        195                 200                 205

Gly Glu Gln Glu Ser Thr Trp Glu Thr Ile Pro His Pro Ser Ile Glu
    210                 215                 220

Glu Phe Lys Tyr Gly Gly Pro Asn Gly Phe Leu Asp Leu Phe Thr Lys
225                 230                 235                 240

Asp Arg Ser Tyr Ala Lys Gln Trp Arg Tyr Thr Asn Ala Pro Asp Ala
                245                 250                 255

Glu Gly Arg Ala Ile Gln Ala Val Tyr Trp Ala Asn Lys Trp Ala Lys
            260                 265                 270

Glu Gln Gly Lys Gly Ser Ala Val Ala Ser Val Val Ser Lys Ala Ala
        275                 280                 285

Lys Met Gly Asp Phe Leu Arg Asn Asp Met Phe Asp Lys Tyr Phe Met
    290                 295                 300

Lys Ile Gly Ala Gln Asp Lys Thr Pro Ala Thr Gly Tyr Asp Ser Ala
305                 310                 315                 320

His Tyr Leu Met Ala Trp Tyr Thr Ala Trp Gly Gly Gly Ile Gly Ala
                325                 330                 335

Ser Trp Ala Trp Lys Ile Gly Cys Ser His Ala His Phe Gly Tyr Gln
            340                 345                 350

Asn Pro Phe Gln Gly Trp Val Ser Ala Thr Gln Ser Asp Phe Ala Pro
        355                 360                 365

Lys Ser Ser Asn Gly Lys Arg Asp Trp Thr Thr Ser Tyr Lys Arg Gln
    370                 375                 380

Leu Glu Phe Tyr Gln Trp Leu Gln Ser Ala Glu Gly Gly Ile Ala Gly
385                 390                 395                 400

Gly Ala Thr Asn Ser Trp Asn Gly Arg Tyr Glu Lys Tyr Pro Ala Gly
                405                 410                 415

Thr Ser Thr Phe Tyr Gly Met Ala Tyr Val Pro His Pro Val Tyr Ala
            420                 425                 430

Asp Pro Gly Ser Asn Gln Trp Phe Gly Phe Gln Ala Trp Ser Met Gln
        435                 440                 445

Arg Val Met Glu Tyr Tyr Leu Glu Thr Gly Asp Ser Ser Val Lys Asn
    450                 455                 460

Leu Ile Lys Lys Trp Val Asp Trp Val Met Ser Glu Ile Lys Leu Tyr
465                 470                 475                 480

Asp Asp Gly Thr Phe Ala Ile Pro Ser Asp Leu Glu Trp Ser Gly Gln
                485                 490                 495

Pro Asp Thr Trp Thr Gly Thr Tyr Thr Gly Asn Pro Asn Leu His Val
            500                 505                 510

Arg Val Thr Ser Tyr Gly Thr Asp Leu Gly Val Ala Gly Ser Leu Ala
        515                 520                 525

Asn Ala Leu Ala Thr Tyr Ala Ala Ala Thr Glu Arg Trp Glu Gly Lys
    530                 535                 540

Leu Asp Thr Lys Ala Arg Asp Met Ala Ala Glu Leu Val Asn Arg Ala
545                 550                 555                 560

Trp Tyr Asn Phe Tyr Cys Ser Glu Gly Lys Gly Val Val Thr Glu Glu
                565                 570                 575

Ala Arg Ala Asp Tyr Lys Arg Phe Phe Glu Gln Glu Val Tyr Val Pro
            580                 585                 590

Ala Gly Trp Ser Gly Thr Met Pro Asn Gly Asp Lys Ile Gln Pro Gly
        595                 600                 605

Ile Lys Phe Ile Asp Ile Arg Thr Lys Tyr Arg Gln Asp Pro Tyr Tyr
    610                 615                 620

Asp Ile Val Tyr Gln Ala Tyr Leu Arg Gly Glu Ala Pro Val Leu Asn
625                 630                 635                 640

Tyr His Arg Phe Trp His Glu Val Asp Leu Ala Val Ala Met Gly Val
                645                 650                 655

Leu Ala Thr Tyr Phe Pro Asp Met Thr Tyr Lys Val Pro Gly Thr Pro
            660                 665                 670

Ser Thr Lys Leu Tyr Gly Asp Val Asn Asp Asp Gly Lys Val Asn Ser
        675                 680                 685

Thr Asp Ala Val Ala Leu Lys Arg Tyr Val Leu Arg Ser Gly Ile Ser
    690                 695                 700

Ile Asn Thr Asp Asn Ala Asp Leu Asn Glu Asp Gly Arg Val Asn Ser
705                 710                 715                 720

Thr Asp Leu Gly Ile Leu Lys Arg Tyr Ile Leu Lys Glu Ile Asp Thr
                725                 730                 735

Leu Pro Tyr Lys Asn
            740

<210>  21
<211>  60
<212>  DNA
<213>  Clostridium thermocellum

<400>  21
gacgttaatc gtgacggtgc gattaactcc tctgacatga ctatattaaa gagatatctg      60

<210>  22
<211>  20
<212>  PRT
<213>  Clostridium thermocellum

<400>  22

Asp Val Asn Arg Asp Gly Ala Ile Asn Ser Ser Asp Met Thr Ile Leu
1               5                   10                  15

Lys Arg Tyr Leu
            20

<210>  23
<211>  159
<212>  DNA
<213>  Clostridium thermocellum

<400>  23
gacgtcaatg atgacggaaa agttaactca actgacgctg tagcattgaa gagatatgtt      60

ttgagatcag gtataagcat caacactgac aatgccgatt tgaatgaaga cggcagagtt     120

aattcaactg acttaggaat tttgaagaga tatattctc                            159

<210>  24
<211>  53
<212>  PRT
<213>  Clostridium thermocellum

<400>  24

Asp Val Asn Asp Asp Gly Lys Val Asn Ser Thr Asp Ala Val Ala Leu
1               5                   10                  15

Lys Arg Tyr Val Leu Arg Ser Gly Ile Ser Ile Asn Thr Asp Asn Ala
            20                  25                  30

Asp Leu Asn Glu Asp Gly Arg Val Asn Ser Thr Asp Leu Gly Ile Leu
        35                  40                  45

Lys Arg Tyr Ile Leu
    50